# EUROPÄISCHE PATENTSCHRIFT

(11) **EP 2 160 458 B1**
(45) Veröffentlichungstag und Bekanntmachung des Hinweises auf die Patenterteilung: **11.07.2018**
(21) Anmeldenummer: 07846568.9
(22) Anmeldetag: 13.11.2007
(51) Int. Cl.: C12M 1/107

(54) **BIOGASANLAGE UND VERFAHREN ZUR ERZEUGUNG VON BIOGAS AUS STROH MIT GÄRRESTPELLETIERUNG**
BIOGAS PLANT AND PROCESS FOR THE PRODUCTION OF BIOGAS FROM STRAW WITH FERMENTATION RESIDUE PELLETING
INSTALLATION DE BIOGAZ ET PROCÉDÉ DE PRODUCTION DE BIOGAZ À PARTIR DE PAILLE AVEC PELLETISATION DES RÉSIDUS DE FERMENTATION

(30) Priorität: 27.06.2007 DE 102007029700; 27.07.2007 WO PCT/EP2007/006681
(43) Veröffentlichungstag der Anmeldung: 10.03.2010
(73) Patentinhaber: Meissner, Jan A., 8700 Küsnacht-ZH (CH)
(72) Erfinder: FELDMANN, Michael, 82349 Krailing-Pentenried (DE)
(74) Vertreter: Schüssler, Andrea
(86) Internationale Anmeldenummer: PCT/EP2007/009809
(87) Internationale Veröffentlichungsnummer: WO 2009/000307

(56) Entgegenhaltungen:
- EP-A- 0 286 100
- EP-A- 0 934 998
- EP-A- 1 681 344
- DE-A1- 4 308 920
- DE-A1- 10 021 383
- DE-A1- 19 857 870
- DE-U1-202004 012 746
- DE-U1-202006 003 293
- DE-U1-202006 003 293
- US-A1- 2006 024 801
- DATABASE WPI Week 200529, Derwent Publications Ltd., London, GB; AN 2005-276902 & JP 2005 087977 A (MITSUI ENG & SHIPBUILDING CO LTD) 07 April 2005

## Beschreibung

Die vorliegende Erfindung betrifft eine Biogasanlage, insbesondere ein Biomassekraftwerk und ein Verfahren zur Biogaserzeugung aus nachwachsenden Rohstoffen.

DE 100 21 383 A1 und DE 198 57 870 A1 betreffen Verfahren und Vorrichtungen zur Aufbereitung von Reststoffgemengen und zur Konversion von kohlenstoffhaltigen Rest- und Rohstoffen in Reststoffgemengen.

EP-A-0 286 100 betrifft ein Verfahren und eine Vorrichtung zur Nutzbarmachung von Bioabfällen.

DE 43 08 920 A1 betrifft eine Vorrichtung zur Behandlung von biologisch abbaubaren Substanzen oder Vorläufern von biologisch abbaubaren Substanzen.

DE 20 2006 003293 U1 beschreibt eine Biogasanlage mit einer Mehrzahl von garagenförmigen Fermentern für eine anaerobe bakterielle Vergärung von Biomasse nach dem Feststoffvergärungsverfahren, jedoch findet keine Dehydrierung und Pelletierung von Gärresten statt.

Die Zusammenfassung der JP 2005 276902 A aus der WPI Datenbank (Database WPI Week 200529, Thomson Scientific, London, GB; AN 2005-276902) beschreibt die Beseitigung von Biomüll unter gleichzeitiger Erzeugung von Biogas.

Traditionell wurden für die Biogaserzeugung biomassehaltige Reststoffe verwendet, z. B. Bioabfall, Speisereste oder Klärschlamm. Diese Substanzen fallen grundsätzlich an, und es ist naheliegend, sie für die Gaserzeugung heranzuziehen. Allerdings ist das Aufkommen solcher Substanzen prinzipiell beschränkt, und daher ist ein weiteres Wachstum der Biogaserzeugung auf Grundlage dieser Substanzen nicht möglich.

Weitere Substanzen, die sich für die Biogaserzeugung eignen, sind Gülle und Festmist, aber auch gezielt angebaute Energiepflanzen, so genannte nachwachsende Rohstoffe. Bei nachwachsenden Rohstoffen, die nur für die Energieerzeugung angebaut werden, besteht jedoch das Problem, dass für ihren Anbau landwirtschaftliche Flächen benötigt werden, die eigentlich für die Nahrungsmittel- und Futtermittelproduktion gebraucht werden. Die Konkurrenz um begrenzte Flächen führt unter anderem zu einem Anstieg der Kosten sowohl für pflanzliche Nahrungsmittel- und Futtermittel als auch für die nachwachsende Rohstoffe (d.h. die Biomasse).

Die meisten gegenwärtig in Deutschland betriebenen Biogasanlagen sind verhältnismäßig klein, befinden sich typischerweise auf einem Bauernhof und werden überwiegend von der eigenen Landwirtschaft mit Gärsubstrat versorgt. In diesem Fall fallen für die Beschaffung der Biomasse nur die Erntekosten an, die der Landwirt leicht kalkulieren kann, und so ist das finanzielle Risiko für die Anschaffung einer Biogasanlage für einen Landwirt vorhersehbar. Finanziell wesentlich riskanter ist es jedoch, größere Biogasanlagen zu errichten, bei denen von vornherein klar ist, dass sie mit zugekaufter Biomasse betrieben werden. In einem solchen Fall kann eine zukünftige Knappheit und damit Verteuerung der Gärsubstrate der gesamten Investition die finanzielle Grundlage entziehen. Ein wesentlicher Gesichtspunkt bei der Planung und Errichtung von größeren Biogasanlagen stellt somit die Sicherheit dar, ausreichend Biomasse für dessen Betrieb zu vorhersagbaren Preisen zur Verfügung zu haben.

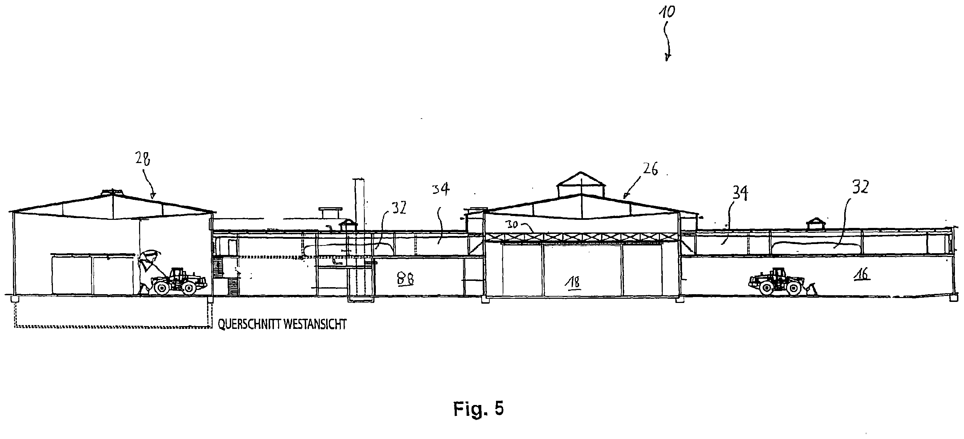

Als etablierte Technik zur Erzeugung von Biogas gelten gegenwärtig die sogenannten Nassfermentationsanlagen, von denen derzeit über 3.500 in Deutschland in Einsatz sind. Diesen stehen lediglich rund 20 Anlagen zur Feststoffvergärung (auch Trockenvergärung genannt) von nachwachsenden Rohstoffen entgegen. Gemäß dem erst kürzlich erschienen Endbericht "Monitoring zur Wirkung des novellierten Erneuerbaren-Energien-Gesetzes (EEG) auf die Entwicklung der Stromerzeugung aus Biomasse", der im Auftrag des Bundesministeriums für Umwelt, Naturschutz und Reaktorsicherheit (BMU) erstellt wurde, hat die Trockenvergärung noch nicht den Stand der Marktreife erreicht. Die in Betrieb befindlichen Anlagen werden dort vielmehr als Demonstrationsanlagen angesehen (siehe Seite 52, Abbildung 5-2 des genannten Endberichts).

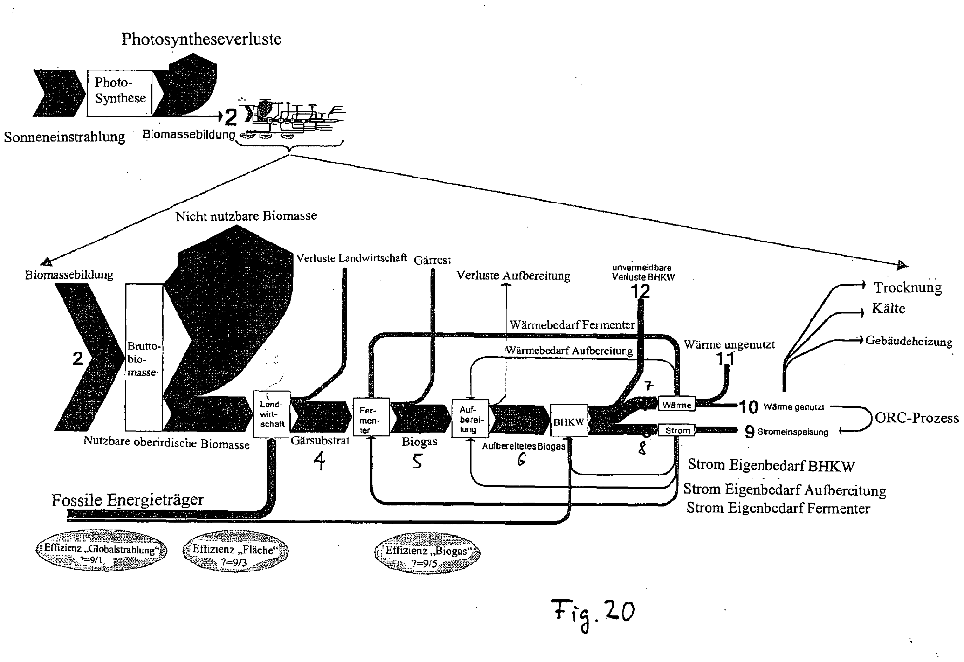

Ein weiterer wichtiger Aspekt neben der sicheren und ausreichenden Beschaffung geeigneter Biomasse ist die effiziente Nutzung derselben. Um die Wirtschaftlichkeit von Biogasanlagen, insbesondere Biomassekraftwerken zu erhöhen, werden gegenwärtig Anstrengungen unternommen, deren Energieeffizienz zu erhöhen. Dem Thema Effizienz in Biogasanlagen widmet sich beispielsweise das Arbeitspapier Nr. 1 mit dem Titel "Biomasse und Effizienz, Vorschläge zur Erhöhung der Energieeffizienz von § 8 und § 7 - Anlagen im Erneuerbaren-Energie-Gesetz", welches im Rahmen des Projekts "Energiebalance - Optimale Systemlösungen für erneuerbare Energie und Energieeffizienz" von Dr. Martin Pehnt und Regine Vogt vom Institut für Energie- und Umweltforschung Heidelberg im Mai 2007 vorgelegt wurde. Diesem Artikel ist die Fig. 20 entnommen, in der der Energiefluss zwischen den unterschiedlichen Komponenten einer Biogasanlage, d.h. Fermenter, Aufbereitungsstelle und BHKW schematisch dargestellt ist. Eine wesentliche Komponente bei der Steigerung der Effizienz ist die Kraftwärmekopplung im BHKW, bei der Abwärme (siehe Pfeil 7 in Fig. 20), die bei der Erzeugung von Strom (siehe Pfeil 8 in Fig. 20) anfällt, genutzt wird, beispielsweise als Fernwärme (10) oder auch um den Fermenter zu erwärmen. Mit herkömmlichen Biomassekraftwerken können von der Energie, die in dem Gärsubstrat (Pfeil 4) enthalten ist, theoretisch 23 bis 52 % nutzbar gemacht werden, in der Praxis sind es jedoch lediglich 10 bis 20 %.

Der Erfindung liegt die Aufgabe zugrunde, eine Biogasanlage, insbesondere ein Biomassekraftwerk sowie ein Verfahren zur Erzeugung von Biogas anzugeben, bei denen sich das Gärsubstrat sicher und wirtschaftlich beschaffen lässt und bei denen die Energie des Gärsubstrats effizient genutzt wird.

Diese Aufgabe wird durch eine Biogasanlage nach Anspruch 1 und ein Verfahren nach Anspruch 8 gelöst. Vorteilhafte Weiterbildungen sind in den abhängigen Ansprüchen angegeben.

Das Verfahren zur Erzeugung von Biogas nach der Erfindung zeichnet sich durch die Kombination von drei wichtigen Aspekten aus, nämlich
1. es wird zumindest teilweise Stroh als Gärsubstrat verwendet,
2. das Stroh wird nach dem Trockenfermentationsverfahren in einem so genannten Garagenfermenter vergoren, und
3. die Gärreste, die aus dem Fermenter entfernt werden, werden dehydriert und pellettiert, um Brennstoffspellets oder Brennstoffbriketts zu erzeugen.

Dabei bezeichnet der Begriff "Stroh" ganz allgemein die getrocknete Halme und Stängel von jeglichen Feldfrüchten, insbesondere Getreidestroh, Rapsstroh, Maisstroh und ähnliche faserhaltige Substrate. Der besondere Vorteil von Stroh ist, dass es sich dabei im Allgemeinen um einen Reststoff handelt, der beispielsweise bei der Getreideproduktion ohnehin anfällt, und daher in ausreichenden Mengen zur Verfügung steht. In der Tat ist Stroh als Gärsubstrat eine der wenigen Ausnahmen zu der oben beschriebenen Konkurrenz zwischen Nahrungsmittelproduktion und Energiepflanzen um die zur Verfügung stehenden Ackerflächen.

Allerdings besteht gegenwärtig ein technisches Vorurteil gegenüber der Verwendung von Stroh als Gärsubstrat. Tatsächlich wird in der Fachwelt gegenwärtig angenommen, dass der Biogasertrag von Stroh für eine ökonomische Biogaserzeugung zu gering ist. Beispielsweise wird in dem Fachartikel "Energetische Verwertung von Stroh - Möglichkeiten und Grenzen" von D. Peisker, T. Hering und Dr. H. Vetter vom Thüringer Landesamt für Landwirtschaft aus dem Februar 2007 die Nutzung von Stroh auf der Basis eines anaeroben bakteriellen Abbaus kategorisch ausgeschlossen (siehe Seite 3 des zitierten Artikels). Der Grund für die Ansicht, dass Stroh sich für die anaerobe Vergärung nicht eigne, liegt an dessen hohen Ligninanteil. Das Lignin, welches für die bei der Gärung beteiligten Bakterien unverdaulich ist, versperrt den Bakterien den Weg zur Zellulose und bewirkt daher, dass der Gärprozess und damit die Biogaserzeugung nur langsam vorankommt. Aus diesem Grund spielt Stroh als Substrat in
der Biogaserzeugung gegenwärtig keine Rolle, und insbesondere in der Nassvergärung gilt es als unbrauchbar.

Im Kontext der vorliegenden Erfindung hat das im Stroh enthaltene Lignin jedoch auch Vorteile. Wenn Stroh als Hauptgärsubstrat verwendet wird, ergibt sich im Zuge der Trockenfermentierung ein Gärrest, der sich aufgrund der faserigen Struktur gut dehydrieren und pelletieren lässt. Dabei hat das in den Gärresten enthaltene Lignin den Vorteil, dass es bei der Pelletierung schmilzt, beim Abkühlen der Pellets wieder erstarrt, und so zu verhältnismäßig festen Pellets oder Briketts führt, die einen wertvollen Brennstoff darstellen.

Selbst wenn man annimmt, dass der Biogasertrag von Stroh geringer ist als derjenige von anderen Gärsubstraten, ist die Energiebilanz des erfindungsgemäßen Verfahrens äußerst günstig, da zusätzlich zu dem erzeugten Strom und der erzeugten Wärme ein wertvoller Brennstoff hergestellt wird. Um auf das Diagramm von Fig. 20 zurückzukommen, konzentrieren sich die gegenwärtigen Bemühungen zur Steigerung der Energieeffizienz auf die Verfahrensschritte und Komponenten jenseits des Pfeils 5 ("Biogas"), d.h. auf die Erzeugung von möglichst viel Biogas aus einer gegebenen Substratmenge und der effizienten Nutzung der im Biogas enthaltenen Energie.

Die vorliegende Erfindung setzt in dem Diagramm von Fig. 20 jedoch einen Schritt früher an, nämlich bei dem Gärsubstrat (Pfeil 4). Der Erfindung liegt die Erkenntnis zugrunde, dass ein wesentlicher Anteil der Energie des Gärsubstrats in Form der Gärreste schlicht verloren geht. In der Darstellung von Fig. 20 werden die Gärreste durch einen verhältnismäßig schmalen Pfeil als Verlust gekennzeichnet. Dies ist im Zusammenhang mit herkömmlichen BMKW zutreffend, weil bei herkömmlichen Verfahren zur Biogaserzeugung die Gärreste lediglich als Dünger verwendet werden, die darin enthaltene Energie jedoch nicht nutzbar gemacht wird.

Im Unterschied hierzu wird bei der vorliegenden Erfindung der Energiegehalt der Gärreste in Form von Brennstoffpellets oder -briketts nutzbar gemacht. Wenn man die Feuerungswärmeleistung der Brennstoffpellets, die aus den Gärresten erzeugt wurden, mitberücksichtigt, ergibt sich selbst bei einem verhältnismäßig geringen Biogasertrag bei der Strohvergärung eine hervorragende Energiebilanz, die sowohl energetisch als ökonomisch äußerst attraktiv ist.

Im Übrigen hat der Erfinder erkannt, dass der Biogasertrag von Stroh entgegen dem allgemeinen Vorurteil nicht grundsätzlich gering sein muss. Der Erfinder hat festgestellt, dass sich im Gegenteil große Mengen an Biogas aus Stroh erhalten lassen, wenn dieses vor der Vergärung chemisch, mechanisch oder thermisch aufgeschlossen wird. Im Folgenden werden eine Vielzahl von Verfahren vorgestellt, mit denen das Stroh aufgeschlossen werden kann und der Gasertrag erheblich gesteigert werden kann.

Ein weiterer besonderer Vorteil der Erfindung besteht darin, dass die Gesamtenergiebilanz verhältnismäßig stabil gegenüber dem relativen Biogasertrag ist, weil der Biogasertrag und der Ertrag an Brennstoffpellets aus Gärresten komplementär zueinander sind. Ein niedrigerer Gasertrag wird also durch einen höheren Ertrag an Brennwert der Gärrestepellets kompensiert und umgekehrt. Der Betreiber der Biogasanlage hat es also in der Hand, durch die Wahl von oder Verzicht auf unterschiedliche Verfahren zum Strohaufschluss den Biogasertrag pro Tonne Trockensubstanz festzulegen. Beispielsweise kann es sich für den Betreiber zu einem gewissen Zeitpunkt rechnen, Biogas mit einem geringeren Wirkungsgrad zu erzeugen und stattdessen den Durchsatz an Stroh durch die Anlage zu erhöhen, beispielsweise weil das Stroh für ihn günstig ist und die Preise für die Gärrestepellets hoch sind. Falls sich die Menge an verfügbarem Stroh verknappt, könnte der Betreiber hingegen durch Verlängerung des Gärzyklus und geeignete Maßnahmen zum Strohaufschluss den relativen Biogasertrag steigern und dadurch mit der gleichen Menge Stroh mehr Biogas und dadurch Strom erzeugen. Auf diese Weise ist der Betreiber bzw. der Investor zu gewissen Grade vor Unwägbarkeiten auf dem Markt für Biomasse geschützt. Obwohl dies ein ökonomisches Problem ist, liefert die vorliegende Erfindung dafür eine technische Lösung, denn der Betreiber hat es zu gewissen Grade in der Hand, wie viel Stroh er kaufen muss, um die Biogasanlage bezüglich des anvisierten absoluten Gasertrags voll auszulasten.

Ein weiterer besonderer technischer Effekt der Kombination von Biogaserzeugung aus Stroh einerseits und Gärrestepelletierung andererseits besteht bei Biogasanlagen, die Generatoren zur Stromerzeugung aufweisen, darin, dass die Abwärme der Generatoren sinnvoll bei der Dehydrierung, insbesondere Trocknung der Gärreste benutzt werden kann. Zwar ist es bereits bekannt, ein Biomassekraftwerk zur Abführung der Abwärme der Generatoren an ein Fern- oder Nahwärmenetz anzuschließen, doch bedeutet dies, dass der Standort für das Kraftwerk durch die Nähe zu einem derartigen Fern- oder Nahwärmenetz stark eingeschränkt ist. Gemäß der Erfindung kann die Abwärme der Generatoren auf vorteilhafte Weise im Zuge der Dehydrierung und Pelletierung der Gärreste verwendet werden. Auch ohne einen Abnehmer für die Wärme zur Verfügung zu haben, kann auf diese Weise die Abwärme der Generatoren sinnvoll genutzt werden.

Die Biogasanlage und die Einrichtung zur Herstellung von Brennstoffpellets können baulich vereinigt oder von einander räumlich getrennt sein.

Vorzugsweise umfasst die Biogasanlage eine Einrichtung zum Auswaschen oder Eluieren der Gärreste. Durch das Auswaschen und Eluieren werden für die Verbrennung schädliche Stoffe wie beispielsweise Chlor, Kalium, Schwefel und Stickstoff aus den Gärresten ausgewaschen. Dies führt zu einer verbesserten Qualität der Brennstoffpellets bzw. -briketts, so dass diese, gegenbenenfalls mit weitergehender Konditionierung, Regelbrennstoffqualität erhalten können. In einer vorteilhaften Ausführungsform wird die Einrichtung zum Auswaschen durch einen thermophil betriebenen Gärrestebunker gebildet, der unten näher beschrieben wird.

Vorzugsweise bestehen mindestens 30 Gewichts-% der Trockensubstanz der Frischmasse aus Stroh, und besonders vorzugsweise mindestens 75 Gewichts-%. Dabei kann es sich um reines Stroh handeln oder um Stroh, welches in Festmist enthalten ist, dessen Trockensubstanz zu ca. 75 % ebenfalls aus Stroh besteht. In einer besonders vorteilhaften Weiterbildung bestehen 30 bis 90 Gewichts-% der Trockensubstanz der Frischmasse aus reinem Stroh, 10 bis 70 Gewichts-% aus Festmist, und 0 bis 30 Gewichts-% aus anderen Substraten. Dadurch wird erreicht, dass die Gärrestepellets zum überwiegenden Anteil aus Stroh bestehen und gut für eine Verfeuerung geeignet sind. Außerdem führt bereits der Harnstoff im Festmist zu einem ganz erheblichen chemischen Aufschluss des Strohs, wenn dieses mit dem Festmist vermischt wird.

Die Einrichtung zum Dehydrieren und Pelletieren der Gärreste umfasst vorzugsweise eine Einrichtung zum mechanischen Dehydrieren desselben, insbesondere eine Schneckenpresse oder einen Dekanter. Mit einer derartigen Schneckenpresse ist es möglich, den Wassergehalt der Gärreste auf beispielsweise 45 % bis 55 % zu reduzieren. Der nach der Behandlung mit der Schneckenpresse verbleibende Wassergehalt ist insbesondere gering genug, dass er unter Verwendung einer Abwärme der vorhandenen Gasmotoren auf einen für die Pelletierung ausreichenden Trockenheitsgrad getrocknet werden können. Ein weiterer Vorteil der mechanischen Dehydrierung besteht darin, dass das Wasser mit darin gelösten für die Verbrennung schädlichen Stoffen wie beispielsweise Chlor, Stickstoff, Schwefel, Kaliumchlorid und/oder Silikate aus den Gärresten ausgepresst wird. Würden die Gärreste nur durch eine thermische Trocknung dehydriert, würde ein weitaus größerer Anteil dieser schädlichen Stoffe in den Gärresten verbleiben.

Vorzugsweise umfasst die Einrichtung zum Dehydrierung und Pelletieren ferner eine Trocknungseinrichtung, bei der es sich vorzugsweise um einen konvektiven Bandtrockner handelt. Wie eingangs erwähnt wird die Trocknungseinrichtung vorzugsweise mit Abwärme von mindestens einem Gasmotor der Biogasanlage betrieben. Vorzugsweise ist der Bandtrockner ein Niedertemperaturbandtrockner, dessen Temperatur 180 °C, vorzugsweise 140 °C nicht übersteigt. Aufgrund der niedrigen Temperatur entstehen bei der Trocknung keine Dioxine und Furane. Mit einem solchen Bandtrockner lässt sich der Wassergehalt der Gärreste auf beispielsweise 7 bis 13 % verringern. Alternativ kann die Trocknungseinrichtung durch einen Trommeltrockner mit Kontakttrocknung gebildet werden, der ebenfalls mit Temperaturen arbeiten kann, die 180 °C, vorzugsweise 140 °C nicht übersteigen.

Vorzugsweise umfasst die Einrichtung zum Dehydrieren und Pelletieren eine Einrichtung zum Zerkleinern der zumindest mechanisch ausgepressten und gegebenenfalls bereits getrockneten Gärreste. Vorzugsweise wird diese Einrichtung durch eine Hammermühle oder eine Strohmühle gebildet.

Vorzugsweise umfasst die Einrichtung zum Dehydrieren und Pelletieren ferner eine Konditionierungseinrichtung, die geeignet ist, den zerkleinerten und zumindest teilweise dehydrierten und/oder getrockneten Gärresten eine oder mehrere der folgenden Zugaben beizumischen: Getreidemehl, Melasse, Branntkalk, Dampf, Holzspäne und/oder Sägemehl. Durch die Beimischung von Getreidemehl und/oder Melasse wird die Festigkeit der Gärrestepellets erhöht. Durch Bedampfung der zu pelletierenden Masse wird sie geschmeidig und lässt sich dadurch besser pelletieren. Eine Beimischung von Brandkalk verbessert das Ascheerweichungsverhalten der Pellets bei deren Verbrennung. Durch das Beimischen von Holzspäne und Sägemehl werden die Brenneigenschaften der Pellets weiter verbessert, insbesondere hinsichtlich Abbrennverhalten, Aschebildung und Emissionen, so dass die Pellets sogar in häuslichen Pelletheizungen verwendbar werden. Außerdem kann den zumindest teilweise dehydrierten und/oder getrockneten Gärresten ein Stickstoff bindendes Mittel, insbesondere ein Stickstoff reduzierendes Reduktionsmittel, Kalkhydrat, Ölpresskuchen, wie sie bei der Gewinnung von Raps- oder Kürbiskernöl entstehen, Kalzium, Magnesium und/oder Aluminium beigemischt werden. Die Ölpresskuchen erhöhen den Brennwert der Pellets, Kalzium, Magnesium und Aluminium können das Brennverhalten verbessern.

Schließlich umfasst die Einrichtung zum Dehydrieren und Pelletieren vorzugsweise eine Pelletierpresse, die geeignet ist, die zerkleinerte und dehydrierte Gärmasse zu Pellets oder Briketts zu pressen.

Vorzugsweise sind zumindest einige der genannten Komponenten der Dehydrier- und Pelletiervorrichtung durch pneumatische Förderanlagen verbunden.

Vorzugsweise umfasst die Einrichtung zum Aufschluss von Stroh eine Einrichtung zur Sattdampfbehandlung. Die Einrichtung zur Sattdampfbehandlung umfasst vorzugsweise einen Druckbehälter und Mittel, die geeignet sind, im Druckbehälter einen Wasserdampf zu erzeugen, mit einem Druck, der zwischen 20 und 30 bar liegt und einer Temperatur, die zwischen 180°C und 250°C liegt. Eine Sattdampfbehandlung findet bei den beschriebenen Drücken und Temperaturen statt und dauert typischerweise 5 bis 15 Minuten. Die Funktion der Sattdampfbehandlung soll am Beispiel von Weizenstroh beschrieben werden.

Weizenstroh besteht zu etwa 40 % aus Cellulose, zu 23 % aus Arabinoxylan (Hemicellulose) und zu 21 % aus Lignin, wobei alle drei Hauptkomponenten eine dicht gepackte Struktur einnehmen. Das wesentliche Hindernis für die biochemische Verwertung der Cellulose und der Hemicellulose ist das für Mikroorganismen unverdauliche Lignin, das den Bakterien den Weg zur Cellulose und zur Hemicellulose versperrt. Bei der Sattdampfbehandlung werden die Ligninstrukturen aufgeweicht bzw. geschmolzen, aber während der verhältnismäßig kurzen Behandlungsdauer im Wesentlichen nicht aus den Halmen ausgelöst. Nach der Sattdampfbehandlung erstarrt das Lignin wieder. Beim Erstarren des Lignins bilden sich jedoch aufgelockerte tröpfchenartige Ligninstrukturen, die ausreichend Zwischenräume lassen, durch die zunächst wässrigen organischen Säuren und dann die Bakterien an die Cellulose und das Arabinoxylan gelangen können, die diese durch die bekannte anaerobe vierstufige Vergärung zersetzen.

Bei der hier beschriebenen Sattdampfbehandlung wird das Lignin also vornehmlich in seiner mikroskopischen Struktur verändert, es wird aber nicht aus den Strohhalmen herausgelöst.

Insbesondere bleibt die Struktur der Strohhalme als solche dabei erhalten. Dies stellt einen Unterschied zur Thermodruckhydrolyse dar, die unter grundsätzlich ähnlichen Bedingungen, jedoch für längere Zeiträume durchgeführt wird, und bei der eine echte Hydrolyse stattfindet, also eine Auflösung vormals fester bzw. trockener Stoffe in Wasser. Durch eine Thermodruckhydrolyse wird die Struktur der Strohhalme aufgelöst, und es ergibt sich eine sirupartige Suspension.

In einer vorteilhaften Weiterbildung umfasst die Einrichtung zum Aufschluss von Stroh einen Behälter zum Einweichen desselben vor der Sattdampfbehandlung, beispielsweise in Wasser. Wenn das eingeweichte Stroh einer Sattdampfbehandlung unterzogen wird, verdampft das eingezogene Wasser schlagartig, wodurch Ligno-Cellulosestrukturen zerreißen und die Cellulose noch besser für die Bakterien zugänglich wird.

In einer vorteilhaften Ausführungsform ist eine Einrichtung zum mechanischen Zerkleinern des Strohs vorgesehen, durch die das Stroh vor der Sattdampfbehandlung mechanisch aufgeschlossen werden kann, beispielsweise durch Häckseln. Dies trägt weiter zum Auflösen der Ligninstrukturen bei und erleichtert die nachfolgende Vergärung.

Alternativ zur Sattdampfbehandlung kann eine Vermahlung beispielsweise in einer Hammermühle erfolgen. Diese Aufschlussform zerstört die Ligninstrukturen mechanisch. Der Erfinder hat festgestellt, dass der Biogasertrag wesentlich mit dem Vermahlungsgrad zunimmt. Tatsächlich ergibt sich in einem Partikelbereich von 2 cm bis 0,1 mm ein Methanertrag, der gegenüber dem Logarithmus der Partikelgröße ungefähr linear abfällt. Vorzugsweise entspricht der Vermahlungsgrad daher mindestens einer Sieblochgröße von 8 mm, besonders vorzugsweise von 1 mm. Der Anteil des Strohs, der zum mechanischen Aufschluss vermahlen werden kann, hängt von dem Vermahlungsgrad ab. Bei einem feinen Vermahlungsgrad, entsprechend einer Sieblochgröße von 1 mm, ist es nicht sinnvoll, das gesamte Stroh zu vermahlen, weil sich sonst ein teigartiger Brei bilden würde, der die Gärmasse verklebt und eine Perkolation verhindert.

In einer alternativen Ausführungsform kann das Stroh auf eine Partikellänge von weniger als 5 cm, vorzugsweise weniger als 2 cm geschnitten und/oder gehäckselt werden. Dies kann beispielsweise schon bei der Ernte geschehen, weil sich mit modernen Ballenpressen Strohballen aus Halmen pressen lassen, die lediglich rund 4 cm lang sind.

Alternativ kann das Stroh jedoch auch in Ballenform aufgeschlossen werden, was insbesondere dessen Transport und Handhabung wesentlich erleichtert, wie unten näher erläutert wird. Da die Struktur beispielsweise von Strohhalmen unter der Sattdampfbehandlung erhalten bleibt, behalten auch Strohballen ihre Form unter der Sattdampfbehandlung bei und können nach dieser einfach und effizient transportiert werden. Ein besonderer Vorteil von Ballen besteht ferner darin, dass diese zuunterst in einen Garagenfermenter geschichtet werden können, wodurch sich die Füllhöhe des Fermenters steigern lässt. Prinzipiell ist die Füllhöhe des Fermenters dadurch begrenzt, dass ab einer gewissen Höhe des Substrats im Fermenter der Druck an dessen Boden so hoch wird, dass das Substrat zu stark verdichtet wird, um Perkolat durchsickern zu lassen. Strohballenschichten, die zuunterst in einen Fermenter eingebracht werden, sind jedoch weitaus druckstabiler als herkömmliches Gärsubstrat. Selbst unter hohem Druck ist die Strohballenschicht noch durchlässig für Perkolat, so dass die übliche Füllhöhe im Fermenter noch auf die Strohballenschicht aufgefüllt werden kann. Die Fermenter können deshalb um die Höhe der Strohballenlage höher konstruiert werden als üblich, was die Fermenter spezifischen Technikkosten (Tor, Gastechnik, Sensorik, Klappen und Öffnungen, Perkolatdüsen, Abläufe, Verrohrung, Pumpen etc.) konstant hält und die Effizienz der Biogasanlage als ganzes erhöht. Vorzugsweise werden die Strohballen aufgelöst, nachdem sie in den Fermenter geschichtet wurden, indem das Garn entfernt wird, mit dem sie zusammengehalten werden.

Um die Effizienz einer Einweichphase und/oder einer Sattdampfbehandlung von Strohballen zu erhöhen, sind in einer vorteilhaften Weiterbildung Mittel zum Perforieren der Ballen vorgesehen. Dabei sind die Mittel zum Perforieren vorzugsweise geeignet, einen Ballen von zwei Seiten so zu perforieren, dass die bei der Perforation von einer Seite entstehenden Löcher und die bei der Perforation von der anderen Seite entstehenden Löcher durch Materialbrücken getrennt sind. Durch eine derartige Perforierung des Ballens werden sowohl das Einweichen des Ballens als auch eine nachfolgende Sattdampfbehandlung effizienter.

In einer vorteilhaften Weiterbildung umfasst die Einrichtung zur Sattdampfbehandlung mindestens einen Spieß, auf den ein Ballen aus Stroh aufspießbar ist, wobei der Spieß einen inneren Hohlraum aufweist, in den Wasserdampf einleitbar ist, und eine Mehrzahl von Öffnungen umfasst, durch die der Wasserdampf aus dem Hohlraum austreten kann. Dadurch kann der unter der Sattdampfbehandlung verwendete heiße und unter hohem Druck befindliche Wasserdampf durch den Spieß in den Ballen eingeführt werden, wodurch erreicht wird, dass die Sattdampf-Atmosphäre auch das Material im Inneren des Ballens in ausreichnendem Maße erreicht. Bei einer einfacheren Version, bei der die Sattdampf-Atmosphäre auf nahe liegende Weise in den Druckbehälter eingeführt würde, kann das Problem auftauchen, dass die in dem Ballen vorhandene Luft in einem inneren Abschnitt des Ballens komprimiert wird, sich aber mit dem Dampf nicht schnell genug durchmischt, so dass die Sattdampfbehandlung in diesem inneren Abschnitt des Ballens weniger wirksam verläuft.

Falls loses Stroh verwendet wird, ist in dem Druckbehälter vorzugsweise ein für Wasserdampf durchlässiger Behälter zur Aufbewahrung derselben vorgesehen. Ferner sind vorzugsweise Mittel zum Transportieren des für Wasserdampf durchlässigen Behälters in und aus dem Druckbehälter vorgesehen, beispielsweise Schienen oder eine Rollenbahn.

In einer besonders vorteilhaften Weiterbildung hat der Druckbehälter eine obere Öffnung, durch die er mit losem Stroh beschickbar ist, und eine untere Öffnung, durch die das lose Stroh aus dem für Wasserdampf durchlässigen Behälter herausfallen kann. Wie unten anhand eines Ausführungsbeispiels näher erläutert wird, kann durch solch einen Aufbau eine "quasi kontinuierliche" Sattdampfbehandlung durchgeführt werden, bei der das lose Stroh chargenweise in den Behälter eingeschüttet wird, der Druckbehälter dann für die Sattdampfbehandlung geschlossen wird, danach das behandelte lose Material durch die untere Öffnung aus dem Behälter fallengelassen wird und schließlich eine nachfolgende Charge durch die obere Öffnung in den Behälter fallengelassen wird.

In einer vorteilhaften Weiterbildung kann die Einrichtung zur Sattdampfbehandlung mehrere Druckbehälter umfassen, die durch Rohrleitungen miteinander verbunden sind. Auf diese Weise kann eine Mehrzahl von Druckbehältern durch eine einzige Dampfquelle, beispielsweise ein einziges Dampfreservoir, versorgt werden, was die Effizienz insbesondere bei größeren Kraftwerken deutlich erhöht.

In einer vorteilhaften Weiterbildung umfasst die Einrichtung zum Aufschluss von Stroh einen Behälter zum Einweichen desselben in einer Wasser-Laugen-Lösung, einer Wasser-Säure-Lösung, Perkolat oder Gülle. Dieses Einweichen stellt ein Beispiel für den eingangs genannten chemischen Aufschluss dar. Dieses Einweichen kann insbesondere nach der Sattdampfbehandlung durchgeführt werden, und zwar sowohl bei losem Stroh wie auch bei ballenförmigem Stroh, wobei die Ballen in diesem Fall vorzugsweise auf die oben beschriebene Weise perforiert sind. Durch dieses Einweichen wird eine (schwach aerobe) Vorhydrolyse initiiert, die vor dem Einbringen in den Fermenter stattfindet. Dazu kann das eingeweichte Stroh nach der Einweichung und vor dem Einbringen in den Fermenter vorzugsweise noch auf 30 bis 50° erwärmt werden. Diese Erwärmung kann mit dem Transport des Strohs von der Einrichtung zum Aufschluss zum Fermenter kombiniert werden, wie unten näher erläutert wird. Durch diese Vorhydrolyse beschleunigt sich die anschließende anaerobe bakterielle Fermentation im Fermenter nochmals. Der Verfahrenschritt des Einweichens des Strohs in einer Wasser-Laugen-Lösung, einer Wasser-Säure-Lösung, Perkolat oder Gülle kann jedoch auch ohne Sattdampfbehandlung verwendet werden.

Man beachte, dass der Aufbau der Biogasanlage und das Verfahren zur Erzeugung von Biogas mit den hier beschriebenen Aufschlussverfahren ohne die Zugabe von zusätzlichen Hefen, Pilzen oder Enzymen vor der Fermentierung im Fermenter auskommt. Tatsächlich wird das ligninhaltige Material allein der Autohydrolyse und der bakteriellen Hydrolyse überlassen. Die Verwendung von Bakterien statt Hefen, Pilzen oder Enzymen ist wesentlich wirtschaftlicher, da die chemischen und biochemischen Prozesse bei der bakteriellen Hydrolyse schneller ablaufen als bei der enzymatischen. Außerdem werden gegenüber dem externen Einsatz von Enzymen und/oder Säuren erhebliche Kosten für deren Beschaffung und Handhabung eingespart. In einer vorteilhaften Weiterbildung werden die Gärreste jedoch in einem Gärrestbunker einer thermophilen Nachgärung unterzogen, bei der die Temperatur mit 50 bis 58 °C höher ist, als im mesophil betriebenen Fermenter. In diesem Nachgärer können dann Enzyme zugefügt werden, die bei den thermophilen Temperaturen gut arbeiten.

Eine wichtige Weiterbildung der Erfindung besteht in der Weise, wie der zusätzliche Verfahrensschritt des Strohaufschlusses in den Betrieb der Biogasanlage integriert wird. Die Einrichtung zur Pelletierung und die Einrichtung zum Aufschluss des Strohs lohnt sich wirtschaftlich vor allem dann, wenn der Durchsatz der Anlage, bzw. des Biomasse-Kraftwerks hoch ist. Gegenwärtig bekannte Biomasse-Kraftwerke zur Feststoffvergärung (Biogasanlagen) sind jedoch im Gegenteil meistens sehr klein und auf den ländlichen Raum beschränkt. Sie verfügen über zwei bis sechs kleinere Fermenter und erreichen eine effektive elektrische Leistung von lediglich 100 bis 700 kW. Dies liegt zum einen daran, dass die Trockenfermentation gemeinhin als noch nicht marktreif angesehen wird, zum andern aber auch an der gestuften Mindestvergütung des EEG für eingespeisten Strom aus Stroh-Anlagen, die bei der Überschreitung einer Grenze von 500 kW um bis zu 15 % abfällt. Außerdem spricht derzeit gegen eine größere Auslegung von Biogasanlagen mit Trockenfermentation, dass der erforderliche Nachschub an Gärsubstrat gemäß den Anforderungen der Financiers über Jahre im voraus gesichert sein muss, und dass sich die Betreiber, bei denen es sich typischerweise um Landwirte handelt, auf die von ihnen selbst erzeugbaren nachwachsenden Rohstoffe verlassen wollen.

Durch die erfindungsgemäße Biogasanlage und das erfindungsgemäße Verfahren, die auch die Verwendung von Stroh wirtschaftlich machen, kann die Versorgung mit Gärsubstrat jedoch auch für weit größere Anlagen sichergestellt werden, da beispielsweise Stroh beim Getreideanbau in weit größeren Mengen anfällt, als es derzeit benötigt wird, und da es sich mit entsprechender (Groß-)Technik auch über größere Strecken verhältnismäßig wirtschaftlich transportieren lässt. Andererseits lohnen sich die Investitions- und Betriebskosten für eine Einrichtung zum Dehydrieren und Pelletieren von Gärresten und für eine Einrichtung zum Aufschließen des Strohs umso mehr, je größer der Durchsatz der Biogasanlage ist. Ein inhaltlicher Zusammenhang zwischen der Pelletierung von Gärresten und der Möglichkeit zum Aufschluss des Strohs einerseits und der Größe der Biogasanlage andererseits besteht insofern, als die Einrichtungen zur Pelletierung von Gärresten und zum Aufschluss des Strohs wesentlich dazu beiträgt, die Versorgung auch größerer Biogasanlagen mit geeignetem Gärsubstrat, nämlich Stroh sicherzustellen, und andererseits die Größe der Biogasanlage und die Verwendung der preisgünstigen Strohs die Investition für die Einrichtungen zur Pelletierung und zum Aufschluss im besonderen und das große Biomasse-Kraftwerk als ganzes erst wirtschaftlich macht.

Bei einer bisher nicht bekannten Größe von Biogasanlagen mit beispielsweise 15 bis 30 großen Garagenfermentem und einer Leistung von über 5 MW, sie sie nachstehend beschrieben ist, muss der Betrieb der Biogasanlage, und insbesondere der Transport des Gärsubstrats und der Gärreste effizient gestaltet werden. Eine weitere Aufgabe besteht darin, die oben beschriebene Gärrestepelletierung und den oben beschriebenen Aufschluss des Strohs in den Betriebsablauf der Biogasanlage zu integrieren.

In einer vorteilhaften Weiterbildung ist die Einrichtung zum chemischen, mechanischen und/oder thermischen Aufschluss von Stroh in einem Anliefer- und Verladebereich der Biogasanlage untergebracht. Der Anliefer- und Verladebereich umfasst vorzugsweise stationäre Fördertechnik, die geeignet ist, Frischmasse aus dem Anliefer- und Verladebereich zu einem Fermentervorplatz zu fördern, von dem aus eine Mehrzahl von Fermentern des Garagentyps zugänglich ist. Während bei herkömmlichen Biogasanlagen mit Garagenfermentem die Frischmasse und die Gärreste komplette mit einem Radlader transportiert werden, ist nach dieser Weiterbildung stationäre Fördertechnik vorgesehen, durch die sich auch große Mengen an Frischmasse effizient zum Fermentervorplatz transportieren lassen, um von dort in die Fermenter eingebracht zu werden. Auch gestattet eine derartige stationäre Fördertechnik es, die gesamte Biogasanlage einzuhausen, wodurch eine Geruchsbelästigung der Umwelt vermieden wird und es möglich wird, die Biogasanlage auch in der Nähe von Wohngebieten zu betreiben.

Wenn die Biogasanlage komplett eingehaust ist, stellt der Anliefer- und Verladebereich gewissermaßen eine Schnittstelle zwischen dem eingehausten Innenbereich der Anlage und dem Außenbereich dar, und ist somit in einem äußeren Abschnitt der Anlage angeordnet. Der Fermentervorplatz hingegen ist aus logistischen Gründen zentral in der Anlage angeordnet. Durch die stationäre Fördertechnik kann die Frischmasse bzw. das Gärsubstrat vom Anliefer- und Verladebereich zum Fermentervorplatz gebracht werden, ohne dass Transportfahrzeuge dazu benötigt würden, die Abgas innerhalb des Einhausungsbereichs erzeugen würden und die außerdem die Betriebskosten erhöhen würden. Vorzugsweise herrscht im Anliefer- und Verladebereich ein leichter Unterdruck, so dass auch beim Anliefern von Frischmasse und beim Verladen von Gärresten nur wenig Luft nach außen dringt, und somit die Geruchsbelästigung minimal ist.

Vorzugsweise befindet sich in dem Anliefer- und Verladebereich mindestens ein eingehauster Anlieferbunker für Frischmasse. Ferner sind vorzugsweise erste Fördermittel vorgesehen, die geeignet sind, Frischmasse aus dem mindestens einen Anlieferbunker für Frischmasse zu einem Frischmassebunker zu befördern. Diese ersten Fördermittel können beispielsweise Förderschnecken, Elevatoren und Förderbänder umfassen, auf denen die Frischmasse aus verschiedenen Anlieferbunkem zum Frischmassebunker befördert werden. Dies hat den Vorteil, dass die Frischmasse bereits dadurch, dass sie aus unterschiedlichen Anlieferbunkern auf ein und denselben Haufen gegeben wird, durchmischt wird, so dass ein späteres Durchmischen der Frischmasse sich erübrigt, bzw. nicht mehr so intensiv vorgenommen werden muss. Bei dieser beschriebenen Frischmasse handelt es sich nicht um das Stroh, das aufgeschlossen werden müsste, sondern um zusätzliche Frischmasse, wie sie in bisher bekannten Biogasanlagen mit Feststoffvergärung verwendet werden.

Ferner umfasst der Anliefer- und Verladebereich vorzugsweise zweite Fördermittel, insbesondere ein Schubschild, welche geeignet sind, die Frischmasse durch den Frischmassebunker hindurch in Richtung auf den Fermentervorplatz zu befördern. Der Frischmassebunker hat dabei eine zweifache Funktion: zum einen dient er als Transportweg vom Anlieferbereich zum Fermentervorplatz, zum anderen dient er als Zwischenspeicher für Frischmasse. Wichtig ist dabei, dass die Frischmasse, die als erstes in den Frischmassebunker eingebracht wurde, diesen auch als erstes verlässt. Das bedeutet, dass die Frischmasse, die auf den Fermentervorplatz geliefert wird, stets etwa gleich alt und damit gleich stark vorhydrolisiert ist. Damit ergibt sich eine Substrat-Konstanz, die für die anschließende Vergärung vorteilhaft ist.

Außerdem umfasst der Anliefer- und Verladebereich in einer vorteilhaften Weiterbildung eine Entladungsstelle für Stroh, und insbesondere für Stroh in Ballenform. In der Entladungsstelle ist vorzugsweise ein Kran vorgesehen, der geeignet ist, Ballenmaterial effizient zu greifen und zu befördern.

In dem Anliefer- und Verladebereich ist vorzugsweise wie oben erwähnt eine Einrichtung zum chemischen, mechanischen und/oder thermischen Aufschluss von Stroh vorgesehen, die von einer der eingangs beschriebenen Arten ist. Insbesondere kann die Einrichtung zum Aufschluss wie oben beschrieben so ausgebildet sein, dass die Ballenform erhalten bleibt, so dass das durch Aufschluss vorbehandelte Stroh in Ballenform von dem Anliefer- und Verladebereich zum Fermentervorplatz transportiert werden kann, was den Transport und die Einbringung in die Fermenter sehr effizient macht.

Vorzugsweise sind dabei dritte Fördermittel, insbesondere Rollenförderer oder Schubförderer vorgesehen, die geeignet sind, einzelne Ballen oder Pakete von Ballen entlang eines Ballenkanals zum Fermentervorplatz zu befördern.

Bei der vorliegenden vorteilhaften Weiterbildung wird also zwischen losem Frischmaterial und Ballenmaterial unterschieden. Auch das Ballenmaterial wird durch die dritten Fördermittel und den Ballen-Kanal auf sehr effiziente Weise von der Peripherie zum Fermentervorplatz transportiert, was einen hohen Durchsatz bei sehr geringen Betriebskosten ermöglicht. Vorzugsweise ist an dem Fermentervorplatz nahen Ende des Ballen-Kanals ein Umsetzer angeordnet, der geeignet ist, Pakete von Ballen aus dem Ballen-Kanal zu entnehmen und an einen Radlader oder Gabelstapler als Paket zu übergeben. Wie unten anhand eines Ausführungsbeispiels näher erläutert wird, ist es vorteilhaft, in einem jeden Fermenter zuunterst eine Schicht aus Ballenmaterial einzubringen. Dies lässt sich nach dieser Weiterbildung der Erfindung wiederum besonders effizient und schnell erledigen, wenn bereits geeignete Pakete von Ballen, beispielsweise Pakete aus acht Ballen, an den Radlader oder Gabelstapler übergeben werden, die dann so, wie sie sind, im Fermenter abgeladen werden können.

Vorzugsweise sind der Anlieferbunker, der Frischmassebunker und/oder der Ballenkanal beheizbar, und vorteilhafterweise mittels Abwärme, welche von einem oder mehreren Gasmotoren erzeugt wird. Durch die Vorerwärmung der Frischmasse werden Temperaturverluste ausgeglichen, die bei der Gärmassenauffrischung am Altmaterial entstehen. Dadurch wird das Wiedereinsetzen der Biogasbildung nach der Gärmassenauffrischung beschleunigt. Ferner wird dadurch die oben beschriebene schwach aerobe Vorhydrolyse möglich, die die Zeit bis zum vollständigen Ausgären der Gärmasse verkürzt und die Anlagenleistung (den Substratdurchsatz) und damit die Wirtschaftlichkeit erhöht.

In einer vorteilhaften Weiterbildung ist ein Gärrestebunker vorgesehen, der vom Fermentervorplatz aus zum Einbringen von Gärresten zugänglich ist. Der Gärrestebunker enthält vorzugsweise stationäre Fördermittel, die geeignet sind, Gärreste durch den Gärrestebunker hindurch abzutransportieren. In einer vorteilhaften Weiterbildung werden diese stationären Fördermittel durch Förderschnecken gebildet, die an den Enden des Gärrestebunkers angeordnet sind. Der Gärrestebunker ist vorzugsweise so bemessen, dass er das zu erwartende Aufkommen an Gärresten von mindestens zwei Tagen fasst.

Der Gärrestebunker nach der genannten Weiterbildung der Erfindung hat eine vierfache Funktion. Erstens dient er als Zwischenspeicher für Gärreste, und zweitens stellt er die Transportvorrichtung für Gärreste aus dem zentralen Fermentervorplatz zur Peripherie bereit. Durch die ausreichende Größe des Gärrestebunkers wird erreicht, dass die Gärreste mindestens zwei Tage zwischengelagert werden können, so dass sie nicht an Wochenenden entnommen werden müssen. Als dritte Funktion findet in dem Gärrestebunker eine Nachvergärung statt, und deshalb ist er an das Biogassystem angeschlossen. So wird aus den Gärresten weiteres Biogas gewonnen, welches bei einer einfacheren Konstruktion verloren ginge. Als vierte Funktion bildet der Gärrestebunker eine Einrichtung zum Auswaschen der Gärreste. Aufgrund des hohen Wassergehalts im Gärrestebunker werden für die Verbrennung schädliche Stoffe wie Chlor, Stickstoff, Schwefel, Kaliumchlorid und Silikate aus den Gärresten ausgewaschen, wodurch die Qualität der Gärrestepellets als Brennstoff erhöht wird.

Am Eintrittsende des Gärrestebunkers ist vorzugsweise ein Schütttrog für Gärreste angeordnet. Die Gärreste können somit direkt vom Fermentervorplatz in den Schütttrog geschüttet werden; sie fließen danach durch die Entnahme am anderen Ende automatisch zur Peripherie, wo sie dehydriert und zu Pellets verarbeitet werden.

In einem einfachen Verfahren zum chemischen Aufschluss von Stroh wird das Stroh bereits Tage vor der Einbringung in den Fermenter mit anderem Frischmaterial, vorzugsweise mit Festmist vermischt. Der im Festmist vorhandene Harnstoff kann dann wiederum das Lignin anlösen und die Cellulose und das Arabinoxylan für die Hydrolyse zugänglich machen. Wichtig ist hierbei, dass zusätzlich bzw. separat bereitgestelltes Stroh durch den im Festmist enthaltenen Harnstoff chemisch aufgeschlossen wird. Die Vermischung des losen Strohs mit dem Festmist würde dabei typischerweise mit größerem zeitlichen Abstand vor der Einbringung in den Fermenter stattfinden, vorzugsweise einige Tage. In einer sehr einfachen Ausgestaltung der Erfindung können Schichten aus Festmist und Schichten aus Stroh ohne zeitlichen Vorlauf abwechselnd im Fermenter aufgebaut werden, wobei gegebenenfalls auch Schichten aus anderen, nicht stark lignifizierten Rohstoffe dazwischen liegen können. Dadurch kann der Harnstoff der oben liegenden Festmistschicht mit dem Perkolat in die Schicht mit dem ligninhaltigen Stroh eindringen und die flächigen Ligninstrukturen zumindest teilweise auflösen. Auch ist es möglich, loses ligninhaltiges Material mit den Gärresten und gegebenenfalls weiteren nachwachsenden Rohstoffen zu vermischen. Dabei sorgt das saure Perkolat dafür, dass sich die flächigen Ligninstrukturen zumindest teilweise auflösen und das Material Biogas liefert, wenn auch nicht mit dem Ertrag, der sich durch die anderen oben beschriebenen Verfahren zum Aufschluss, insbesondere der Sattdampfbehandlung und/oder der Vermahlung, erzielen lassen.

Vorzugsweise wird das Animpfmaterial, das zusammen mit der Frischmasse wieder in den Fermenter kommt, vor der Vermischung mittels einer mechanischen Presse, wie beispielsweise einer Schneckenpresse, durchgewalkt und ausgepresst, wodurch das Material zumindest teilweise mechanisch aufgeschlossen wird und außerdem gegebenenfalls noch eingebundene Nährstoffe für die anaerobe, bakterielle Fermentation zugänglich gemacht werden. Die Schneckenpresse kann dabei mobil ausgestaltet sein, beispielsweise auf einem Tieflader angeordnet sein, so dass sie auf dem Fermentervorplatz zu dem betreffenden Fermenter gefahren werden kann.

In einer vorteilhaften Weiterbildung ist ferner eine Einrichtung zur Thermodruckhydrolyse vorgesehen, mit der das Material sowohl vor als auch nach Durchlauf der Feststoffvergärungsanlage aber vor der Gärrestentsorgung einer Thermodruckhydrolyse unterzogen werden kann. Die Thermodruckhydrolyse findet unter ähnlichen Bedingungen statt, wie die eingangs beschriebene Sattdampfbehandlung, jedoch für eine längere Zeitdauer von beispielsweise 60 bis 120 Minuten. Bei der Thermodruckhydrolyse wird das Lignin vollständig ausgelöst, so dass sich eine sirupartige Suspension bildet. Außerdem findet eine echte Hydrolyse, das heißt, eine Aufspaltung von Polymeren in Monomere durch physikalische Einwirkung von Wasser und Hitze statt. Die sirupartige Suspension kann dann in den Fermentationsprozess (zurück-) gegeben werden oder an Unternehmen verkauft werden, die aus diesem Material Kraftstoffe der zweiten Generation herstellen. Dabei wird auch der Ligninanteil verwertet sowie weitere, unverwertete organische Substanz. Dies ist bei einer anaeroben, bakteriellen Vergärung nicht möglich.

Zum besseren Verständnis der vorliegenden Erfindung wird im Folgenden auf das in den Zeichnungen dargestellte bevorzugte Ausführungsbeispiel Bezug genommen, das anhand spezifischer Terminologie beschrieben ist. Es sei jedoch darauf hingewiesen, dass der Schutzumfang der Erfindung dadurch nicht eingeschränkt werden soll, da derartige Veränderungen und weitere Modifizierungen an der gezeigten Biogasanlage und dem gezeigten Verfahren sowie weitere Anwendungen der Erfindung, wie sie darin aufgezeigt sind, als übliches derzeitiges oder künftiges Fachwissen eines zuständigen Fachmanns angesehen werden. Die Figuren zeigen Ausführungsbeispiele der Erfindung, nämlich:
- Fig. 1: einen Aufriss eines Biomassekraftwerks nach einer Weiterbildung der Erfindung von Westen betrachtet,
- Fig. 2: einen Aufriss des Biomassekraftwerks von Fig. 1 von Norden betrachten,
- Fig. 3: einen Aufriss des Biomassekraftwerks von Fig. 1 von Süden betrachtet,
- Fig. 4: einen Aufriss des Biomassekraftwerks von Fig. 1 von Osten betrachtet,
- Fig. 5: eine Querschnittsansicht des Biomassekraftwerks von Fig. 1 in westlicher Ansicht,
- Fig. 6: einen Grundriss des Erdgeschosses des Biomassekraftwerks von Fig. 1,
- Fig. 7: einen vergrößerten Ausschnitt aus dem Grundriss von Fig. 6, der eine Strom- und Wärmeerzeugungsanlage zeigt,
- Fig. 8: einen vergrößerten Ausschnitt aus der Grundrissdarstellung von Fig. 6, der einen Anliefer- und Verladebereich zeigt,
- Fig. 9: einen Grundriss des Obergeschosses des Biomassekraftwerks von Fig. 1,
- Fig. 10: eine schematische Darstellung zweier Ansichten eines perforierten Strohballens,
- Fig. 11: eine schematische Querschnittsansicht einer Einrichtung zur Sattdampfbehandlung,
- Fig. 12: eine schematische Querschnittsansicht einer weiteren Einrichtung zur Sattdampfbehandlung, die für loses ligninhaltiges Material bestimmt ist,
- Fig. 13: eine schematische Querschnittsansicht einer Einrichtung zur Sattdampfbehandlung, die zur Sattdampfbehandlung von ballenförmigem Material bestimmt ist,
- Fig. 14: eine schematische Querschnittsansicht einer Einrichtung zur Sattdampfbehandlung, die eine Mehrzahl von Druckbehältern umfasst,
- Fig. 15: ein Blockdiagramm wesentlicher Komponenten einer Einrichtung zum Dehydrieren und Pelletierung von Gärresten,
- Fig. 16 bis Fig. 19: zeigen Tabellen, in denen unterschiedliche Szenarien des Biogas-, Wärme- und Brennstoffpelletertrags in Abhängigkeit von dem relativen Biogasertrag für das Biomassekraftwerk von Fig. 1 bis 9 aufgelistet sind,
- Fig. 20: zeigt ein schematisches Diagramm zur Energieeffizienz einer Biogasanlage, das dem Artikel "Biomasse und Effizienz, Vorschläge zur Erhöhung der Energieeffizienz von § 8 und § 7 - Anlagen im Erneuerbaren-Energie-Gesetz" entnommen ist, und
- Fig. 21: zeigt ein detailliertes Energieflussdiagram für eines der in Tabellen 16 bis 19 gezeigten Szenarien.

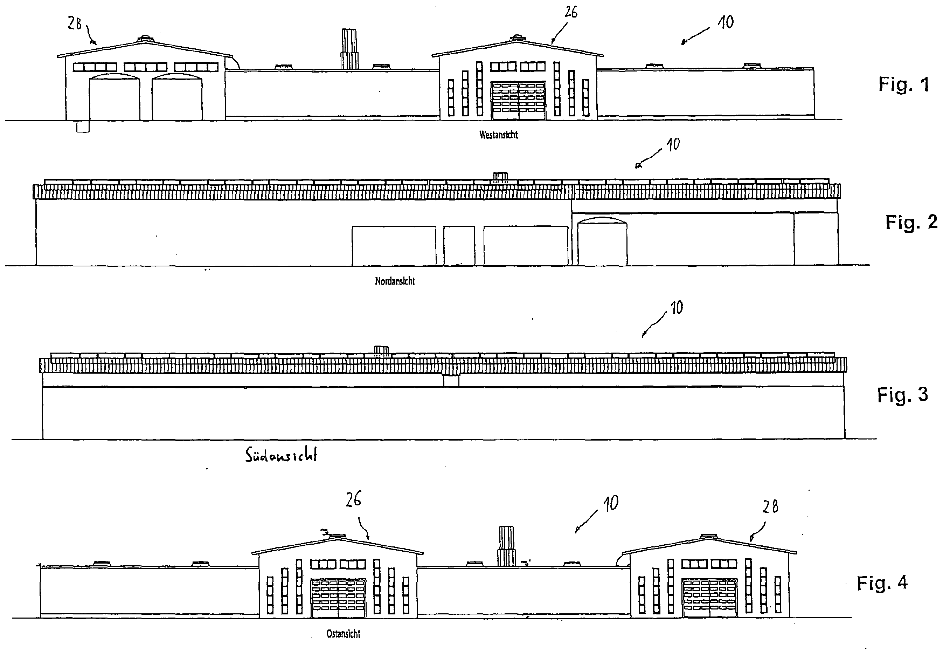

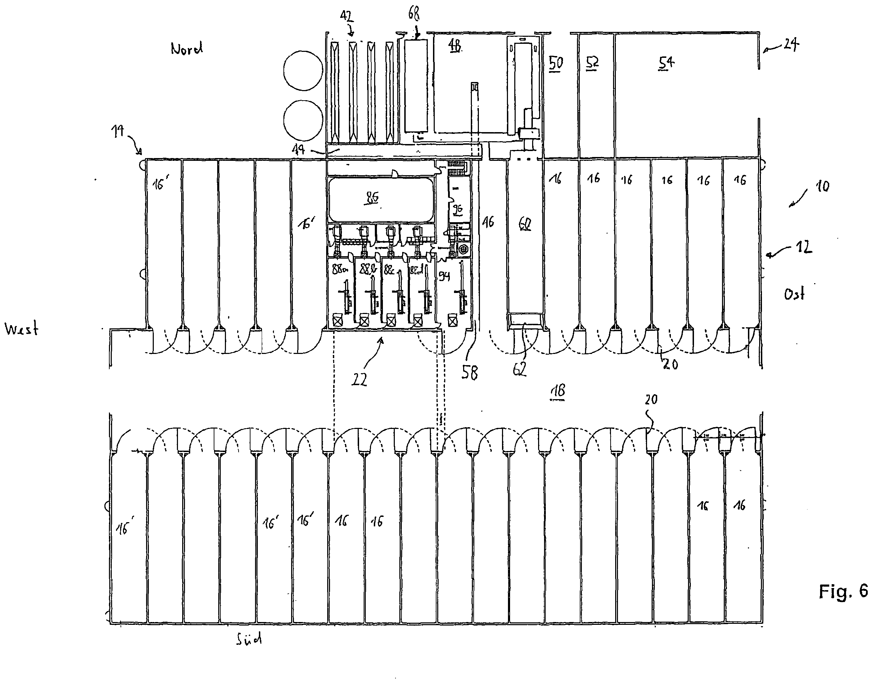

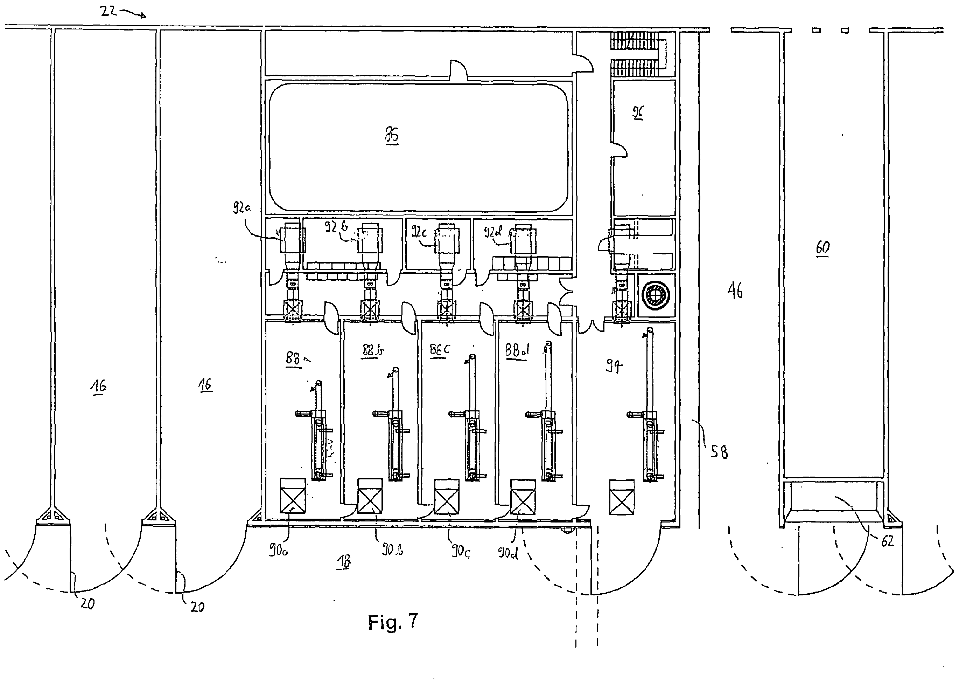

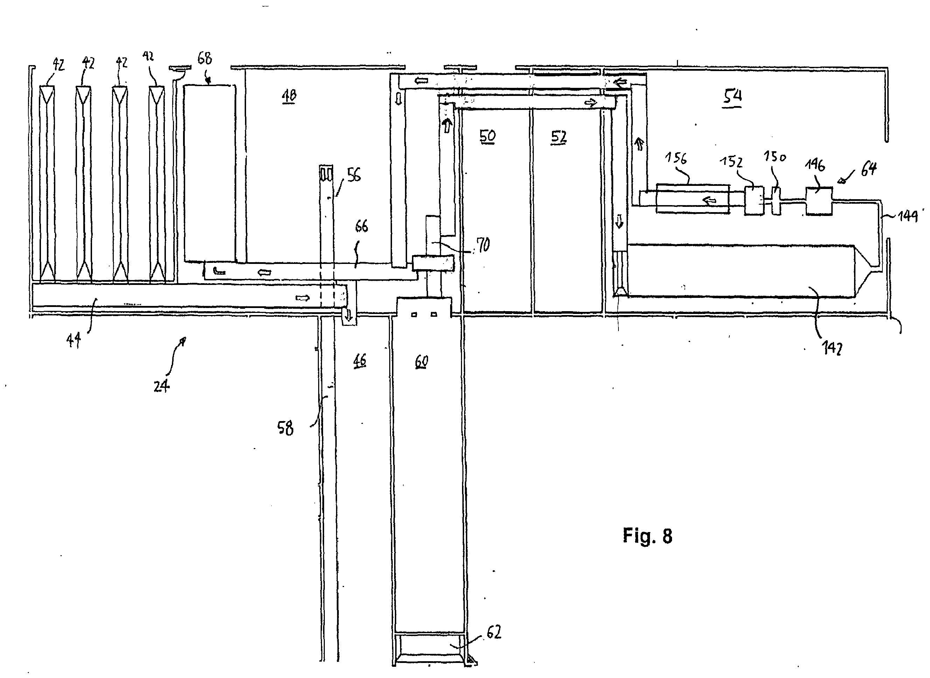

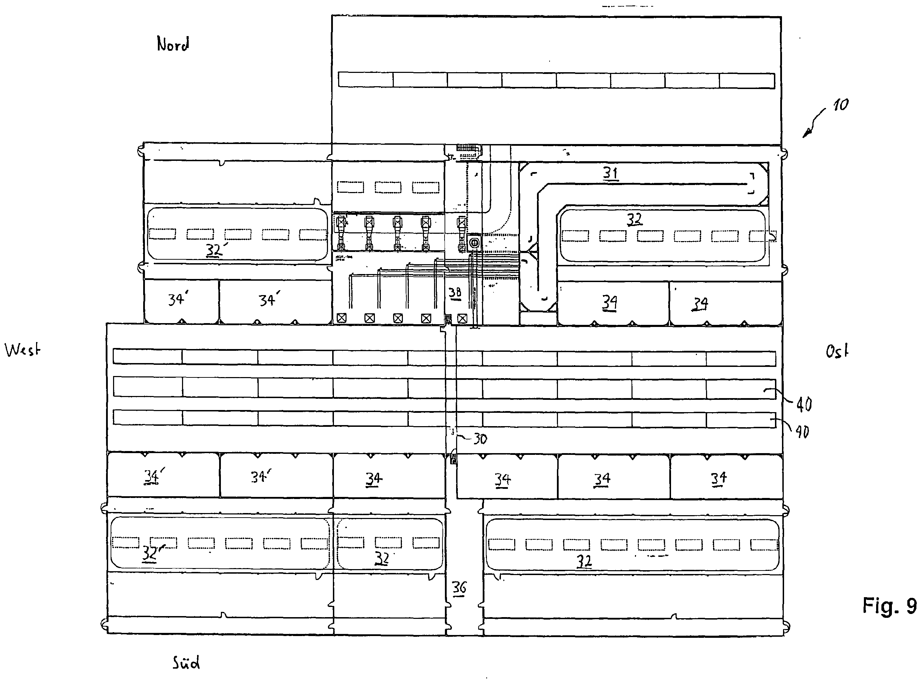

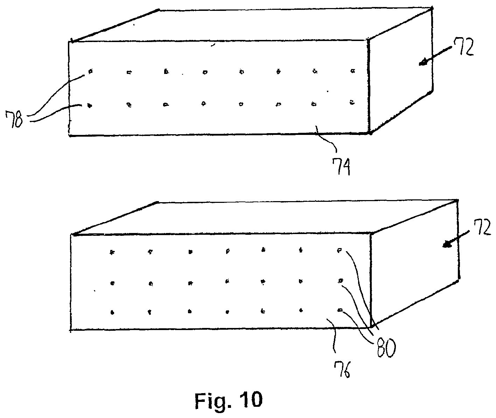

Im Folgenden wird ein Biomassekraftwerk (BMKW) 10 als Ausführungsbeispiel einer Biogasanlage nach einer Weiterbildung der Erfindung im Detail beschrieben. Dabei zeigen die Fig. 1 bis 4 vier Außenansichten des BMKW 10 und Fig. 5 einen Querschnitt desselben. In Fig. 6 ist der Grundriss des Erdgeschosses des BMKW 10 gezeigt. Fig. 7 zeigt einen vergrößerten Teilbereich des Grundrisses von Fig. 10, in dem eine Strom- und Wärmeerzeugungsanlage des BMKW gezeigt ist. Fig. 8 zeigt einen anderen Teilausschnitt des Grundrisses von Fig. 6, in dem ein Anliefer- und Verladebereich vergrößert dargestellt ist. Fig. 9 zeigt eine Grundrissansicht des Obergeschosses des BMKW 10.

Unter Bezugnahme auf den Grundriss von Fig. 6 gliedert sich das BMKW 10 in einen Grundabschnitt 12 und einen Ausbauabschnitt 14. Der Grundabschnitt 12 umfasst 18 Fermenter des Garagentyps, die in zwei Reihen angeordnet sind, in der Darstellung von Fig. 5 in einer nördlichen und einer südlichen Reihe. Zwischen den beiden Reihen von Fermentern 16 befindet sich ein Fermentervorplatz 18, zu dem sich die Tore 20 der Fermenter 16 öffnen. Man beachte, dass der Übersicht halber nicht alle Fermenter 16 und Fermentertore 20 in den Figuren mit Bezugszeichen versehen sind.

Ferner umfasst der Grundabschnitt 12 eine Strom- und Wärmeerzeugungsanlage 22, die in Fig. 7 vergrößert dargestellt und unten näher im Detail beschrieben wird. Außerdem umfasst der Grundabschnitt 12 einen Anliefer- und Verladebereich 24, der in Fig. 8 vergrößert dargestellt ist und ebenfalls unten näher beschrieben wird.

Wie den Fig. 1 bis 6 zu entnehmen ist, ist der gesamte Grundabschnitt 12 durch eine Hallenstruktur eingehaust, zu der insbesondere ein Fermentervorplatz (FVP)-Hallenabschnitt 26 und ein Anliefer- und Verladebereichshallenabschnitt 28 gehört, wie insbesondere in Fig. 1, 4 und 5 gut zu erkennen ist. Die gesamte Hallenkonstruktion oder Einhausung des Grundabschnitts 12 wird durch eine große zentrale Entlüftungsanlage entlüftet, so dass im Inneren der Hallenkonstruktion stets ein leichter Unterdruck gegenüber dem Atmosphärendruck herrscht.

Der Ausbauabschnitt 14 besteht im Wesentlichen aus 11 zusätzlichen Fermentern 16' und einer Verlängerung des FVP-Hallenabschnitts 26. Der Ausbauabschnitt 14 dient dazu, bei Bedarf bis zu 11 zusätzliche Fermenter 16' vorzusehen. Das bedeutet, dass das BMKW 10 zunächst ohne den Ausbauabschnitt 14 gebaut und in Betrieb genommen würde. Im Betrieb stellt sich dann heraus, ob die vorhandenen 18 Fermenter 16 des Grundabschnitts 12 ausreichend Biogas erzeugen, um die vier Gasmotoren (nicht gezeigt), die für das BMKW 10 bestimmt sind, unter Volllast mit Gas zu versorgen. Falls dies nicht der Fall ist, kann die entsprechende Anzahl Fermenter 16' im Ausbauabschnitt 14 ergänzt werden, der somit auch kleiner ausfallen kann als in Fig. 6 gezeigt. Mit anderen Worten hat das BMKW 10 einen modularen Aufbau, der zum Erreichen einer optimalen Endkonfiguration vorteilhaft ist, weil der exakte Biogasertrag von einer Vielzahl von Faktoren, darunter die Beschaffenheit der vorhandenen Frischmasse, abhängt und nicht theoretisch exakt vorhersagbar ist.

Die nördliche und die südliche Fermenterreihe sind durch eine Technikbrücke 30 verbunden, die insbesondere in Fig. 5, 6 und 9 zu sehen ist. Die Technikbrücke 30 überspannt den FVP 18 in einer Höhe, die gestattet, dass Radlader, von denen in Fig. 5 zwei exemplarisch gezeigt sind, unter ihr selbst mit voll ausgefahrener Ladeschaufel hindurch fahren können, ohne dass sie die Technikbrücke berühren und beschädigen können.

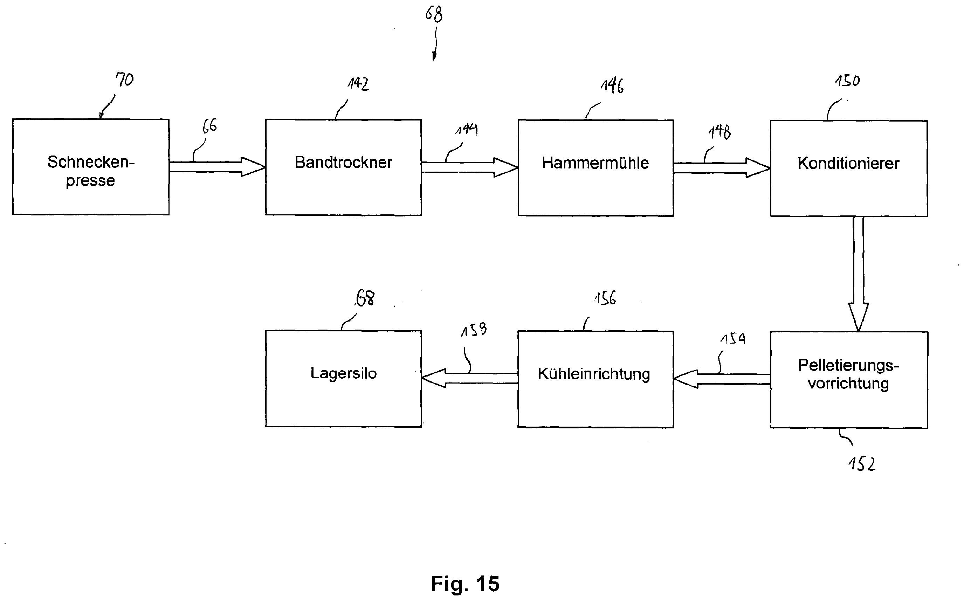

Unter Bezugnahme auf Fig. 9 umfasst das Obergeschoß des BMKW 10 drei FolienGasspeicher 32 im Grundabschnitt 12 und zwei weitere Foliengasspeicher 32' im Ausbauabschnitt 14. Die Foliengasspeicher 32 sind in den Querschnittsansichten von Fig. 5 und 15 gut zu erkennen. Sie nehmen auf nachfolgend näher beschriebene Weise das Biogas auf, welches in den Fermentern 16 bzw. 16' erzeugt wird.

Ferner umfasst das Obergeschoß fünf Perkolatumlauftanks (PUT) 34 im Grundabschnitt 12 und vier PUTs 34' im Ausbauabschnitt 14, die ebenfalls in den Querschnittsansichten von Fig. 5 gut zu sehen sind. Ein PUT 34 ist jeweils oberhalb von drei Fermentern 16 angeordnet und empfängt von ihnen Perkolat, welches am Boden der Fermenter gesammelt und in den PUT 34 gepumpt wird. Mit dem Begriff "Perkolat" bezeichnet man den flüssigen, gülleähnlichen Bestandteil der Gärsubstanz.

Ferner befindet sich im Obergeschoß ein Abgas-Abkühlraum 31, ein südlicher Technikraum 36 und ein nördlicher Technikraum 38, die durch die Technikbrücke 30 miteinander verbunden sind. Im FVP-Hallenabschnitt 26 und im Anliefer- und Verladebereichshallenabschnitt 28 sind ferner Lichtbänder 40 angeordnet.

Nachdem ein grober Überblick über die Bestandteile des BMKW 10 gegeben wurde, werden im folgenden die einzelnen Abschnitte und Komponenten sowie deren Betriebsweise im Detail beschrieben.

### 1. Fermentervorplatz

Der Fermentervorplatz (FVP) 18 ist im Zentrum den BMKW 10 angeordnet. Er dient als Transportweg für Frischmasse zu den jeweiligen Fermentern 16, 16' bzw. von Gärrestesubstanz aus den Fermentern 16, 16'. Außerdem dient er als Anmischfläche, auf der der Inhalt eines Fermenters ausgebreitet wird, von dem etwa ein Fünftel bis ein Viertel als Gärrest entnommen wird und zum Ausgleich dieser Entnahme und des durch die Vergasung entstehenden Masseverlusts etwa ein Drittel durch Frischmasse ergänzt und mit der alten Gärmasse vermischt wird. Diese Arbeit kann auf dem FVP 18 von einem großen Radlader verrichtet werden, wie er in Fig. 5 schematisch dargestellt ist. In der Mitte des FVPs 18 befindet sich eine mit einem Gitterrost versehene Ablaufrinne, in die Sickersäfte und freigesetztes Perkolat fließen. Auf der Höhe der Technikbrücke 30 hat die Ablaufrinne einen Sammelschacht (nicht gezeigt) aus dem die anfallenden Flüssigkeiten über eine Perkolat-Ringleitung (nicht gezeigt) zu einem der PUTs 34 gefördert werden.

### 2. Anliefer- und Verladungsbereich

Der Anliefer- und Verladungsbereich 24 ist in Fig. 8 im Grundriss vergrößert dargestellt. Im gezeigten Ausführungsbeispiel wird, was die Anlieferung betrifft, zwischen loser Frischmasse und Strukturfrischmasse bzw. Ballenfrischmasse unterschieden. Für die lose Frischmasse sind in der gezeigten Ausführungsform vier Anlieferbunker 42 vorgesehen, die durch den Anliefer- und Verladebereichshallenabschnitt 28 eingehaust sind. Ein LKW kann rückwärts in den eingehausten Anlieferbunker rangieren und die Frischmasseladung dort in die Anlieferbunker 42 abkippen oder abschieben. Da im gesamten Anliefer- und Verladebereich 24 ein leichter Unterdruck herrscht, treten kaum störende Gerüche aus der Einhausung nach außen aus. Jeder Anlieferbunker 42 hat einen nach unten konisch zulaufenden Boden, an dessen tiefster Stelle eine oder mehrere Doppelförderschnecke (nicht gezeigt) vorgesehen sind, die die Frischmasse horizontal bis zu einem Becher-Elevator (nicht gezeigt) fördert, welcher die Frischmasse auf ein Förderband 44 befördert, oder direkt auf ein tiefergelegenes Förderband.

Von dem Förderband 44 wird die Frischmasse in einen Frischmassebunker 46 fallengelassen. Da die Frischmasse aus vier oder mehr verschiedenen Bunkern auf demselben Förderband 44 befördert wird und auf denselben Haufen in dem Frischmassebunker 46 geworfen wird, wird die Frischmasse automatisch durchmischt.

Der Frischmassebunker 46 ist eine längliche Kammer, die den Anliefer- und Verladebereich 24 mit dem Fermentervorplatz 18 verbindet, wie insbesondere in Fig. 6 zu sehen ist. Der Frischmassebunker 46 hat eine Fußbodenheizung, mit der die Frischmasse bereits auf eine Temperatur von 42°C erwärmt wird, damit die Gärmasse innerhalb eines Fermenters 16, 16', welche durch die Frischmasse ergänzt wird, durch diese nicht abgekühlt wird, so dass der Gärprozess nach Verschließen des Fermenters 16 wieder schnell startet und ggf. auch bereits eine schwach aerobe Vorhydrolyse stattfinden kann, die die Gärzeit verkürzt und die Anlagenleistung (Durchsatz an Gärsubstrat) sowie die Wirtschaftlichkeit der Anlage erhöht.

Der Frischmassebunker 46 hat eine dreifache Funktion. Zum einen dient er als Zwischen- oder Pufferspeicher für lose Frischmasse. Zum anderen dient er als Transportweg zwischen dem Anliefer- und Verladebereich 24, also der Peripherie des BMKWs 10, und dem zentral gelegenen Fermentervorplatz 18. Zum Transport ist in dem Frischmassebunker 46 ein Schubschild bzw. Schieber angeordnet (nicht gezeigt) der jeweils neu von oben eingeschüttete lose Frischmasse in Richtung auf den Fermentervorplatz 18 schiebt. Dann wird der Schieber zurückgefahren, um Platz für neue Frischmasse zu machen. Durch diesen Schiebemechanismus wird erreicht, dass die Frischmasse ungefähr in der gleichen Reihenfolge, in der sie am Eingang in den Frischmassebunker 46 eingeworfen wurde, auch auf der Seite des Fermentervorplatzes 18 aus ihm herausgeschoben wird. Dies bedeutet, dass die Frischmasse, die zum Fermentervorplatz 18 gelangt, immer ungefähr gleich alt und daher von konstanter Beschaffenheit ist, was für die anschließende Vergärung vorteilhaft ist. Schließlich findet im Frischmassebunker eine schwach aerobe Vorhydrolyse statt, bei der in der Frischmasse enthaltene Säuren beginnen, autohydrolytisch zu wirken.

Ferner umfasst der Anliefer- und Verladebereich 24 einen Abschnitt für das Anliefern und Transportieren von Struktur- bzw. Ballenmaterial, insbesondere für Stroh. Dieser Abschnitt zum Anliefern und Transportieren von Ballenmaterial umfasst einen Bereitstellungsraum 48, einen Ballen-Anlieferungsraum 50, einen Aufschlussbereich 52 und ein Zwischenlager 54. Im folgenden wird dieser Bereich des Anliefer- und Verladebereichs 24 unter Bezugnahme auf Stroh als stark lignifiziertes, ballenförmiges Strukturmaterial beschrieben, aber es versteht sich, dass dieser Abschnitt auch zur Anlieferung, Bearbeitung und den Weitertransport von anderem ballenförmigen Strukturmaterial verwendet werden kann.

Ein Kran (nicht gezeigt) ist an einer Laufschiene so angebracht, dass er Strohballen in jedem der Räume 48 bis 54 aufgreifen und ablegen kann. Die Strohballen werden im Strohanlieferungsraum 50 angeliefert und von dem Kran (nicht gezeigt) effizient in das Zwischenlager 54 transportiert. Bevor das Stroh zum Fermentervorplatz 18 befördert wird, wird es in dem Aufschlussbereich 52 vorbehandelt, nämlich aufgeschlossen. Der Aufschluss des Strohs ist nötig, da das Stroh stark lignifiziert ist, und die Bakterien im Fermenter 16 aufgrund der Lignin krustierten Cellulose nur sehr schlecht an die vom Lignin eingeschlossenen Nährstoffe gelangen. In dem Aufschlussbereich 52 kann das Stroh je nach Ausführungsform des BMKW 10 auf unterschiedliche Arten aufgeschlossen werden. Beispielsweise kann das Stroh chemisch aufgeschlossen werden, indem es in einem Behälter eingeweicht wird, welcher Wasser, eine Wasser-Laugen-Lösung oder eine Wasser-Säure-Lösung enthält. Durch die Einweichung wird das Lignin, das die Cellulose weitgehend eingeschlossen hat, teilweise aufgelöst. Nach der Entnahme aus dem Behälter ist die Cellulose nicht mehr hinter einer Ligninkruste verborgen, sondern für die Hydrolyse und die Bakterien zugänglich. Dadurch wird das Stroh, welches in herkömmlichen Nass- oder Trockenvergärungsanlagen bisher nur als Strukturmaterial verwendet wird, zu einem wertvollen Gärsubstrat, welches zur Biogasentwicklung wesentlich beiträgt.

In einer alternativen Ausführungsform kann das Stroh in dem Aufschlussbereich 52 aber auch auf andere Weise aufgeschlossen werden, beispielsweise mechanisch unter Verwendung einer Hammermühle oder Strohmühle oder indem es einem Thermodruck ausgesetzt wird, d.h. bei hohem Druck von beispielsweise 20 bis 30 bar für fünf bis zehn Minuten auf 180°C bis 250°C erhitzt wird. Dabei weicht das Lignin auf. Nach dem Erkalten des Strohs erstarrt das Lignin zwar wieder, jedoch in Form sehr kleiner Kügelchen mit Zwischenräumen dazwischen, die den Weg für die autohydrolytischen, organischen Säuren und für die anaeroben Bakterien zu den Nährstoffen des Strohs freigeben. Ein weiteres Ausführungsbeispiel ist eine Erweiterung der Thermodruckbehandlung, bei der der Druck in dem entsprechenden Behälter plötzlich reduziert wird, wodurch das Wasser in den Strohstrukturen verdampft und sich sehr rasch ausdehnt. Dabei werden die Ligninstrukturen zerrissen und die Nährstoffe für die anaeroben Bakterien freigelegt. Die weiteren Details des Strohaufschlusses werden im nachfolgenden Abschnitt angegeben.

Im Bereitstellungsraum 48 ist ein Rollenförderer 56 vorgesehen, auf den einzelne Strohballen und/oder Strohballenpakete durch den Kran (nicht gezeigt) aufgelegt werden, und der die Strohballen durch einen Strohkanal 58, der parallel zum Frischmassebunker 46 angeordnet ist, zum Fermentervorplatz 18 befördert (siehe Fig. 6).

Wie der obigen Beschreibung zu entnehmen ist, wird sowohl die lose Frischmasse als auch die ballenförmige Frischmasse durch stationäre Fördertechnik von dem Anliefer- und Verladebereich 24 zum Fermentervorplatz 18 befördert. Insbesondere stellen dabei der Frischmassebunker 46 und der Strohkanal 58 die Verbindung zwischen dem zentralen Fermentervorplatz 18 und dem peripheren Anliefer- und Verladebereich 24 bereit, und dieser Transport geschieht vollständig im eingehausten BMKW 10. Der Transport mit der stationären Fördertechnik ist für hohe Durchsätze geeignet und insbesondere schneller, platzsparender und kostengünstiger als eine Anlieferung mit Radladern. Wie Fig. 6 zu entnehmen ist, enden der Strohkanal 58 und der Frischmassebunker 46 an einer zentralen Stelle des Fermentervorplatzes 18, so dass die Wege zwischen dem fermentervorplatzseitigen Ende des Frischmassebunkers 46 bzw. Strohkanals 58 und dem zu beliefernden Fermenter 16 im allgemeinen kurz sind.

Wie oben erwähnt wurde, ermöglicht der Aufschluss des Strohs im Aufschlussbereich 52 es, Stroh trotz seines hohen Ligningehaltes als Gärsubstrat zu verwenden. Dies ist äußerst vorteilhaft, weil Stroh bei der Getreideproduktion ohnehin anfällt, und gar nicht ausreichend Verwendung für dieses besteht. Da das BMKW 10 für nachwachsende Rohstoffe konzipiert ist, bietet es sich an, in der Umgebung des BMKW 10 speziell zur Verwendung im BMKW 10 geeignete Rohstoffe anzubauen, die jedoch im allgemeinen nicht für die Ernärung oder als Futtermittel bestimmt sind. Dies stellt jedoch einen gewissen Ziel-Konflikt dar, weil stets ein bestimmter Anteil von den begrenzten zur Verfügung stehenden Flächen für die Nahrungsproduktion reserviert ist. Die Verwendung von Stroh als Gärsubstrat stellt eine sehr attraktive Lösung dar, da Stroh, welches bei der Getreideproduktion als Reststoff anfällt, die gleichzeitige Produktion von Nahrungsmitteln und kraftwerkstauglicher Biomasse erlaubt. Außerdem lassen sich aus strohhaltigen Gärresten wertvolle Brennstoffpellets erzeugen, wie unten näher erläutert wird.

Stroh hat aber noch einen weiteren Vorteil. Im Allgemeinen ist die Füllhöhe in Fermentern durch den Druck begrenzt, der sich am Fermenterboden ergibt: Dieser Druck muss stets so niedrig sein, dass das Gärsubstrat noch durchlässig für Perkolat ist. Wenn man hingegen, gemäß einer Weiterbildung der Erfindung, in der untersten Lage eines jeden Fermenters 16 eine Schicht aus Strohballen einbringt, kann auf diese Schicht noch die gesamte übliche Füllhöhe an Gärsubstanz geschichtet werden, da die Strohballenschicht auch bei dem dann auftretenden Druck noch durchlässig für Perkolat ist. Diese unterste Strohschicht stellt also eine zusätzliche Menge an Gärsubstrat dar, die in einem Fermenter verwendet werden kann, so dass die Anlagenleistung (Raumleistung gemessen in neuem Substrat pro Fermenter und Tag) erheblich gesteigert wird.

In einer vorteilhaften Weiterbildung der Erfindung werden die Strohballen in Paketen aus acht Strohballen auf den Rollenförderer 56 aufgelegt, die zwei Ballen breit und vier Ballen hoch sind. Diese Pakete werden als Ganzes durch den Strohkanal 58 transportiert und an dessen Ende am Fermentervorplatz 18 von einem Umsetzer (nicht gezeigt) abgehoben und an einen Radlader oder Gabelstapler übergeben, der die Pakete ebenfalls als Ganzes oder in 2 Teilen empfängt und zum Fermenter bringt. Aus diesen Paketen kann relativ einfach und zügig die besagte unterste Strohballenschicht aufgebaut werden.

Wie weiter in Fig. 8 zu sehen ist, ist ein Gärrestebunker 60 vorgesehen, der sich parallel zum Frischmassebunker zwischen dem Fermentervorplatz 18 und dem Anliefer- und Verladebereich 24 erstreckt. Der Gärrestebunker 60 hat an seinem dem Fermentervorplatz zugewandten Ende ein Schütttrog 62 für Gärreste, die den Eingang in den Gärrestebunker 60 bildet. In diesen Schütttrog 62 werden Gärreste von einem Radlader gekippt. Von dort werden sie mittels einer Förderschnecke in den Gärrestebunker 60 gedrückt. Durch das diskontinuierliche Nachdrücken immer neuer Gärreste wird die Masse langsam durch den Gärrestebunker 60 bis zum anderen Ende befördert, wo sie durch weitere Förderschnecken aus dem Gärrestebunker 60 transportiert werden.

Der Gärrestebunker 60 hat eine vierfache Funktion. Zum einen dient er als Transportweg zwischen dem Fermentervorplatz 18 im Zentrum des BMKW 10 und dem Anliefer- und Verladebereich, mit ähnlichen Vorteilen, wie sie im Hinblick auf den Frischmassebunker 46 und den Strohkanal 58 beschrieben wurden. Zum anderen dient der Gärrestebunker 60 aber auch als thermophil betriebener Nachgärer und wirkt quasi wie ein weiterer Fermenter. Daher ist der Gärrestebunker 60 auch an das Biogassystem angeschlossen. Drittens dient der Gärrestebunker 60 als Zwischenspeicher für Gärreste. Er ist so dimensioniert, dass er mindestens soviel Gärreste fasst, wie an zwei Tagen anfallen können. Als vierte Funktion bildet der Gärrestebunker eine Einrichtung zum Auswaschen der Gärreste. Aufgrund des hohen Wassergehalts im Gärrestebunker werden für die Verbrennung schädliche Stoffe wie Chlor, Stickstoff, Schwefel, Kaliumchlorid und Silikate aus den Gärresten ausgewaschen, wodurch die Qualität der Gärrestepellets als Brennstoff erhöht wird.

An den Gärrestebunker schließt ein Förderband 66 an, mit dem Gärreste erst zu einer Schneckenpresse 70 und dann zu einer Dehydrierungs- und Pelletierungseinrichtung 64 befördert werden, die unten gesondert beschrieben wird. Mit der Schneckenpresse 70 wird ein erheblicher Teil des Wassers aus den Gärresten ausgepresst. Zusammen mit dem Wasser wird ein erheblicher Teil der oben genannten für die Verbrennung schädlichen Stoffe aus den Gärresten ausgepresst. Dies stellt einen erheblichen Vorteil gegenüber einem Verfahren dar, bei dem die Gärreste nur thermisch getrocknet würden, weil in dem Fall wesentlich mehr von den schädlichen Substanzen in den Gärresten verbleiben würden. Die ausgepresste flüssige Phase wird als Prozesswasser in den Gärrestebunker 60 zurückgeführt. In einer vorteilhaften Weiterbildung wird es davor chemisch aufbereitet, um die genannten für die Verbrennung schädlichen Substanzen zumindest teilweise zu entfernen. In einer weiteren vorteilhaften Weiterbildung wird ein Teil der flüssigen Phase entsorgt, wodurch ebenfalls eine Schadstoffabfuhr stattfindet.

### 3. Strohaufschluss

Wie eingangs erwähnt, wird bei dem gezeigten Biomasse-Kraftwerk das Stroh im Anliefer- und Verladebereich 24 angeliefert und im Aufschlussbereich 52 und eventuell zusätzlich im Bereitstellungsraum 48 aufgeschlossen. In dieser Ausführungsform der Erfindung wird Stroh in Ballenform angeliefert und auch in Ballenform aufgeschlossen, um schließlich in Ballenform in die Garagenfermenter 16 eingebracht zu werden. Die Ballen haben dabei vorzugsweise eine Dichte von über 200 kg/m³, die sich nur mit Höchstdruckballenpressen erzeugen lassen (konventionelle Strohballen haben eine Dichte von 130 - 160 kg/m³). Der Vorteil einer solch hohen Dichte der Strohballen besteht darin, dass dann die Kapazität eines LKW sowohl bezüglich des zulässigen Gewichtes der Ladung als auch des möglichen Volumens der Ladung optimal ausgeschöpft wird, so dass das Stroh auch über weitere Strecken zu wirtschaftlichen Bedingungen geliefert werden kann.

In der hier gezeigten Ausführungsform umfasst die Vorbehandlung zum Aufschluss des Strohs vier Schritte, die in dem Aufschlussbereich 52 bzw. dem Bereitstellungsraum 48 durchgeführt werden, nämlich
1.) Perforieren bzw. Lochen der Strohballen,
2.) Einweichen der Strohballen in Wasser,
3.) Sattdampfbehandlung der eingeweichten Strohballen, und
4.) Einweichen der Strohballen in Perkolat.

Diese Schritte und die dabei eingesetzten Vorrichtungen werden im Folgenden beschrieben.

Parallel kann ein Teil des Strohs in die Aufschlussform "Vermahlung" und/oder in die Aufschlussform "Thermodruckhydrolyse" gegeben werden. Die Kombination der verschiedenen Aufschlussformen ist besonders vorteilhaft, denn jede hat ihre Vor- und Nachteile für den Praxisbetrieb. Durch die Kombination wird der beste Gesamteffekt erzielt.

Beispielsweise ist es vorteilhaft, unabhängig von der Vorbehandlung des übrigen Strohs, einen Teil des Strohs zu vermahlen, insbesondere zu einer Pulverkonsistenz, und der übrigen Frischmasse beizumischen. Das vermahlene Stroh führt zu einem besonders hohen Gasertrag, jedoch ist der Anteil im Verhältnis zur Frischmasse insofern begrenzt, als das durch Perkolat benetzte pulverisierte Stroh eine klebrige Masse bildet, die wegen der Handhabbarkeit mit ausreichend lockerer Frischmasse gemischt werden muss.

Vorzugsweise besteht zwischen 5 und 25 Gewichtsprozent des Gärsubstrates als ganzes aus gemahlenem Stroh. Vorzugsweise werden zwischen 5% und 35% des insgesamt verwendeten Strohs vermahlen und dadurch mechanisch aufgeschlossen.

Ferner ist es vorteilhaft, unabhängig von der Vorbehandlung des übrigen Strohs 5-20% des insgesamt verwendeten Strohs in einer Thermodruckhydrolyse aufzuschließen und das dabei erhaltene, sirupähnliche Material, das so genannte "slurry" in den Perkolatkreislauf einzubringen.

Schließlich ist es vorteilhaft, unabhängig von den übrigen Verfahrensschritten zum Strohaufschluss das aus dem Fermenter entnommene Gärsubstrat mechanisch durchzupressen.

### 3.1. Perforation

Die Perforation der Strohballen dient dazu, das Innere des Strohballens für das Einweichen, für die Sattdampfbehandlung und für die nachfolgende Einweichung in Perkolat zugänglich zu machen. In der hier beschriebenen Ausführungsform werden die Strohballen von zwei Seiten gelocht, wie unter Bezugnahme auf Fig. 10 näher erläutert wird.

Fig. 10 zeigt oben eine perspektivische Ansicht eines Strohballens 72, in der auf dessen Unterseite 74 geblickt wird. Die untere Abbildung zeigt eine perspektivische Ansicht desselben Strohballens 72, bei der auf dessen Oberseite 76 geblickt wird. Von der Unterseite 74 aus ist der Strohballen 72 durch einen ersten Satz Löcher 78 perforiert, die nicht durch den gesamten Ballen 72 hindurchgehen. Ferner ist der Strohballen 72 von der Oberseite 76 aus mit einem zweiten Satz Löcher 80 perforiert, die ebenfalls nicht durch den gesamten Strohballen 72 hindurchgehen. Die Löcher 78 und 80 sind so gegeneinander versetzt, dass die Löcher des ersten Satzes 78 und die Löcher des zweiten Satzes 80 durch Materialbrücken voneinander getrennt sind. Durch diese Art der Lochung kann das Einweichwasser des zweiten Schritts, der Sattdampf des dritten Schritts und das Perkolat des vierten Schritts gut in das Innere des Strohballens 72 eindringen, ohne auf der anderen Seite wieder herauszulaufen.

### 3.2. Einweichen

In dem Aufschlussraum 52 von Fig. 8 sind geeignete Behälter zum Einweichen von Strohballen vorgesehen, die in der Darstellung nicht gezeigt sind. Die Größe der Behälter zum Einweichen sind auf die Ausmaße der Strohballen abgestimmt, so dass das Einweichen Platz sparend und effizient durchgeführt werden kann.

### 3.3. Sattdampfbehandlung

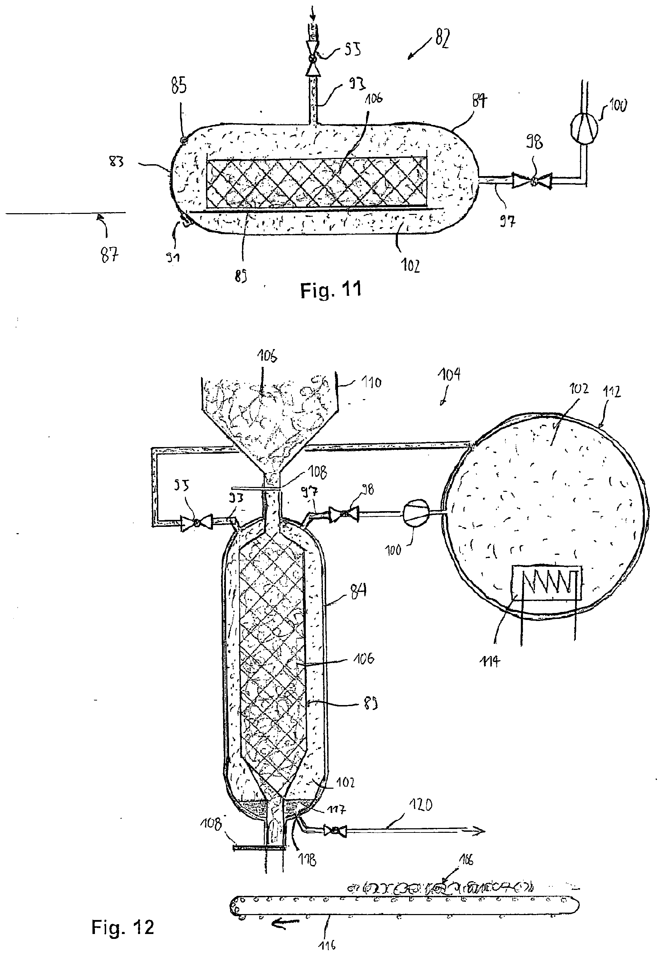

Im Aufschlussbereich 52 oder im Bereitstellungsraum 48 ist eine Einrichtung zur Sattdampfbehandlung vorgesehen. Unter Bezugnahme auf Fig. 11 bis 14 werden verschiedene Einrichtungen zur Sattdampfbehandlung beschrieben, die in der gezeigten Anlage oder einer abgewandelten Anlage verwendet werden können.

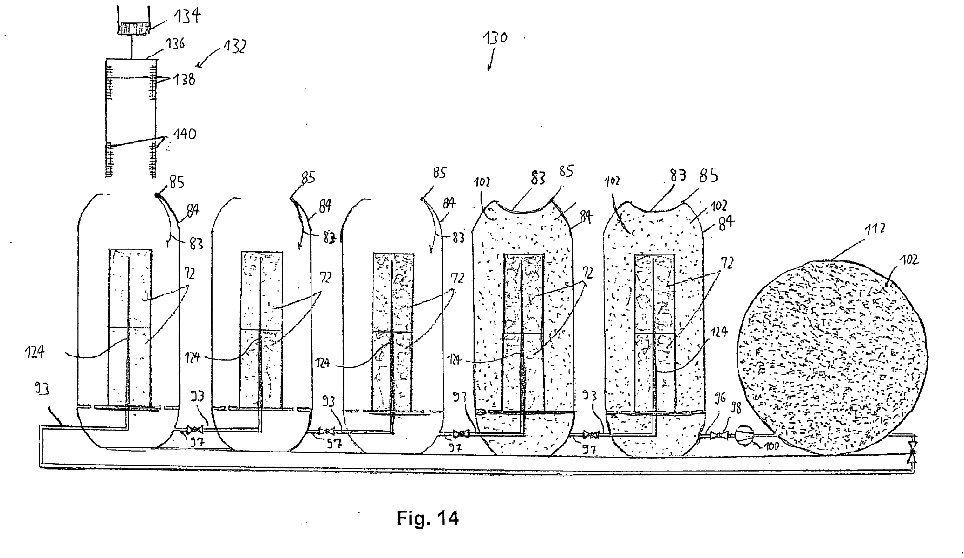

Fig. 11 zeigt in einer schematischen Querschnittsansicht einen einfachen Aufbau einer Einrichtung 82 zur Sattdampfbehandlung. Die Einrichtung 82 umfasst einen Druckbehälter 84 mit einem Deckel 83, der über ein Gelenk 85 schwenkbar am Druckbehälter 84 angelenkt ist. Eine Zuführeinrichtung für das Stroh ist schematisch angedeutet und durch Bezugszeichen 87 bezeichnet. Falls mit der Einrichtung 82 zur Sattdampfbehandlung Strohballen behandelt werden sollen, kann die Zuführeinrichtung 87 beispielsweise durch ein Förderband oder einen Rollenförderer gebildet werden. Falls die Einrichtung 82 für loses Stroh 106, verwendet werden soll, kann die Zuführeinrichtung 87 aus Schienen bestehen, auf denen ein Behälter 89 für loses Material in den Druckbehälter 84 geschoben werden kann. Der Behälter 89 ist für Wasserdampf durchlässig, aber geeignet, das lose Material zu halten und kann beispielsweise ein oben offener Gitterbehälter bzw. Korb sein. Der Deckel 83 des Druckbehälters 84 kann durch einen Schließmechanismus 91 verschlossen werden. Vorzugweise wird der Deckel 83 zum Öffnen des Druckbehälters 84 nach innen geschwenkt, wie dies beispielsweise in Fig. 14 zu sehen ist, so dass der Deckel 83 durch den Druck im Inneren des Druckbehälters 84 in die geschlossene Stellung gedrückt wird und dadurch einfacher abgedichtet wird.

Der Druckbehälter 84 ist mit einer Zufuhrleitung 93 und einem Zuführungs-Ventil 95 verbunden, durch die Sattdampf 102 mit einem Druck von bis zu 30 bar und einer Temperatur von bis zu 250°C von einem Dampfreservoir (nicht gezeigt) in den Druckbehälter 84 eingeführt werden kann. Ferner ist der Druckbehälter 84 mit einer Auslassleitung 97 mit einem Auslass-Ventil 98 verbunden, über die der Dampf nach der Sattdampfbehandlung aus dem Druckbehälter 84 ausgelassen werden kann. Ferner ist in der Auslassleitung 97 ein Kompressor 100 angeordnet, mit dem der Sattdampf 102 in das Reservoir (nicht gezeigt) zurückbefördert werden kann.

Im Folgenden wird das Verfahren der Sattdampfbehandlung unter Bezugnahme auf die Einrichtung 82 zur Sattdampfbehandlung von Fig. 11 erläutert. Zunächst wird das ligninhaltige Material 106 als Ballen oder als loses Material in einem Behälter wie in dem Behälter 89 in den Druckbehälter 84 eingebracht und der Druckbehälter 84 geschlossen. Danach wird das Ventil 95 in der Zufuhrleitung 93 geöffnet, so dass heißer Wasserdampf mit einer Temperatur von 180°C bis 250°C und einem hohen Druck von zwischen 20 und 30 bar von einem Dampfreservoir (nicht gezeigt) in den Druckbehälter 84 eingeführt wird. Der eingeführte Sattdampf ist in Fig. 11 schematisch angedeutet und mit dem Bezugszeichen 102 bezeichnet.

Das ligninhaltige Material 106 wird dem Sattdampf 102 für 5 bis 15 Minuten ausgesetzt. Dabei wird das Lignin in dem Material geschmolzen, aber nicht aus dem Material ausgelöst. Für die Effizienz der Sattdampfbehandlung ist es vorteilhaft, wenn das Material, z.B. das Stroh, in dem oben genannten zweiten Schritt zuvor eingeweicht wurde, weil das Wasser dann bereits im Material vorhanden ist und nur noch erhitzt werden muss, was die Behandlungsdauer verkürzt.

Nach der vorbestimmten Verweildauer von 5 bis 15 Minuten wird der Sattdampf durch die Auslassleitung 97 aus dem Druckbehälter 84 entlassen. Dieses Entlassen des Drucks findet vorzugsweise schlagartig statt, so dass der Druck innerhalb von 5 Sekunden oder weniger um mindestens 80 % abnimmt. Durch die rasche Absenkung des Drucks verdampft das Wasser in den Strukturen des ligninhaltigen Materials schlagartig und dehnt sich dabei rasch aus. Dabei werden die Ligninstrukturen des Strohs zerrissen, so dass die Nährstoffe (Cellulose und Arabinoxylan) für wässrige organische Säuren und die anaeroben Bakterien zugänglich werden.

Nach dem Entlassen des Drucks aus dem Druckbehälter 84 wird das ligninhaltige Material 106 aus dem Druckbehälter 84 entfernt, und es kühlt sich ab. Beim Abkühlen erhärtet das geschmolzene Lignin wieder. Jedoch bilden sich beim Erstarren des Lignins nicht wieder die ursprünglichen flächigen Strukturen, sondern es koaguliert vielmehr in einer Tröpfchenstruktur, die Zwischenräume lässt, durch die zunächst organische Säuren und dann Bakterien an die Cellulose und das Arabinoxylan (Hemicellulose) gelangen können.

Der in Fig. 11 grundsätzlich gezeigte Aufbau der Einrichtung 82 zur Sattdampfbehandlung kann auf vielfältige Weise modifiziert werden, und einige Beispiele solcher Modifikationen werden im Folgenden angegeben. Dabei werden identische oder funktionell gleiche Komponenten mit denselben Bezugszeichen wie in Fig. 11 bezeichnet, und deren Beschreibung nicht wiederholt.

Fig. 12 zeigt einen Aufbau einer Einrichtung zur Sattdampfbehandlung, die für eine quasi kontinuierliche Verarbeitung von losem Material ausgebildet ist. Auch hier ist im Inneren des Druckbehälters 84 ein Behälter 89 für loses Material 106 vorgesehen, jedoch ist dieser in dem Druckbehälter 84 fest installiert. Zum Befüllen des Behälters 89 wird ein druckfester Schieber 108 geöffnet, so dass das ligninhaltige Material 106 aus einem Trichter 110 in den Behälter 88 fällt. Wenn eine ausreichende Menge Material 106 im Behälter 89 ist, wird der druckfeste Schieber 108 geschlossen, und die Sattdampfbehandlung findet auf dieselbe Weise statt, wie sie unter Bezugnahme auf Fig. 11 beschrieben wurde. Zusätzlich zu den Komponenten von Fig. 11 ist jedoch in Fig. 12 ein Reservoir 112 für Sattdampf 102 gezeigt, welches über eine Heizung 114 verfügt. Nach der Sattdampfbehandlung wird der Dampf über die Auslassleitung 97 entlassen und über den Kompressor 100 in das Reservoir 112 gedrückt. Danach wird ein weiterer druckfester Schieber 108 am unteren Ende des Behälters geöffnet, und das aufgeschlossene lose Material 106 fällt auf ein Förderband 116 zum Weitertransport.

Am unteren Ende des Behälters 84 sammelt sich insbesondere bei der Thermodruckhydrolyse eine flüssige Masse 117, die man als "Slurry" bezeichnet und die über eine weitere Leitung 118 abgelassen und über eine Leitung 120 in die Perkolatumlauftanks (nicht gezeigt) geleitet wird.

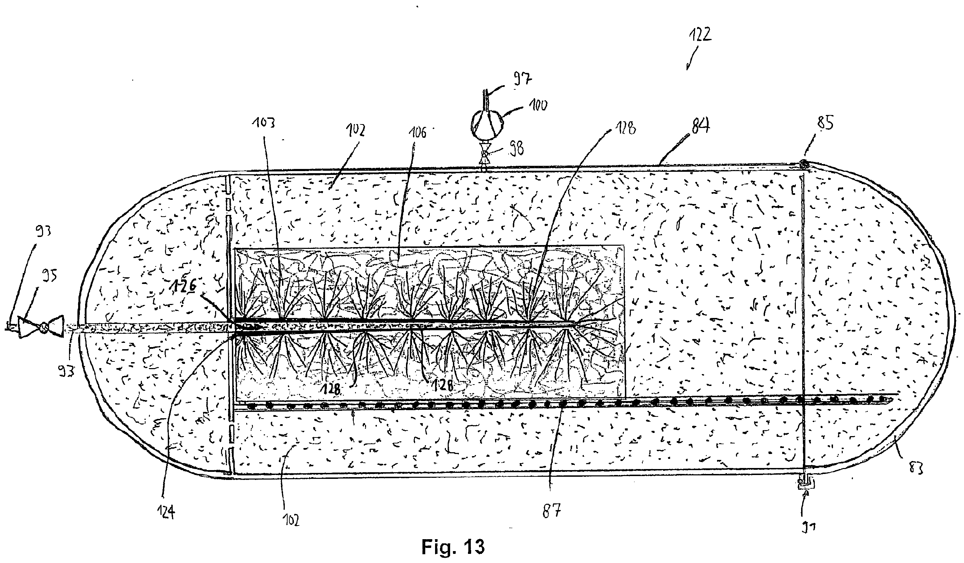

Fig. 13 zeigt eine weitere Ausführungsform 122 einer Einrichtung zur Sattdampfbehandlung, die speziell für die Behandlung von Ballenmaterial, insbesondere Strohballen 72, ausgelegt ist. Der Aufbau ist grundsätzlich ähnlich zum Aufbau von Fig. 11 und wird nicht erneut beschrieben. Der wesentliche Unterschied besteht jedoch darin, dass ein Spieß oder Dorn 124 vorgesehen ist, der einen inneren Hohlraum 126 und mit diesem inneren Hohlraum 126 verbundene düsenartige Öffnungen 128 hat. Der innere Hohlraum 126 ist mit der Zufuhrleitung 93 in Fluidverbindung.

Im Betrieb der Einrichtung zur Sattdampfbehandlung 122 von Fig. 13 wird ein Strohballen 72 bzw. 106 über die Zuführeinrichtung 87, die in der gezeigten Ausführungsform durch eine Rollenbahn gebildet wird, in der Darstellung von Fig. 13 von rechts in den Druckbehälter 84 eingebracht und auf den Spieß 124 aufgespießt. Dann wird der Druckbehälter 84 wie gehabt verschlossen, und der Sattdampf 102 wird über die Zufuhrleitung 93, den inneren Hohlraum 126 des Spießes 124 und die düsenartigen Öffnungen 128 in den Strohballen 72 injiziert. Dadurch wird erreicht, dass auch das Innere des Strohballens 72 wirksam mit dem Sattdampf in Kontakt kommt. Wird nämlich der Sattdampf wie in Fig. 11 gezeigt lediglich von außen an das Material zugeführt, kann es passieren, dass insbesondere bei einem sehr stark gepressten Ballen 72 der Sattdampf nicht ausreichend mit dem Material im Inneren des Ballens in Kontakt kommt. Statt dessen wird die Luft, die sich im Ballen befindet, durch den unter hohem Druck befindlichen Dampf im Inneren des Ballens komprimiert, möglicherweise ohne sich bei den verhältnismäßig kurzen Behandlungszeiten ausreichend mit dem heißen Dampf zu durchmischen. Durch die Verwendung des Spießes 124 wird eine gründliche Sattdampfbehandlung auch im Inneren des Ballens 72 gewährleistet.

Schließlich zeigt Fig. 14 eine weitere Einrichtung 130 zur Sattdampfbehandlung, die fünf Druckbehälter 84 umfasst, die ähnlich wie bei der Einrichtung 122 von Fig. 13 mit Spießen 124 versehen sind. Allerdings sind bei der Einrichtung 130 von Fig. 14 die Druckbehälter 84 vertikal angeordnet, so dass die Strohballen von oben mit einem Kran 132 in die Druckbehälter eingeführt werden können 84. Der Kran 132 umfasst eine Laufkatze 134 und ein Gestell 136, an dem ein oberer Greifer 138 für einen oberen Ballen und ein unterer Greifer 140 für einen unteren Ballen ausgebildet sind. Mit dem Kran 130 können somit zwei vertikal übereinander angeordnete Strohballen 72 gegriffen werden, von oben in den Druckbehälter 84 eingeführt und auf einen Spieß 124 aufgespießt werden, der zu diesem Zwecke etwa doppelt so lang ausgebildet ist wie der Spieß 124 von Fig. 13.

Sämtliche Druckbehälter 84 von Fig. 14 sind über eine Rohrleitung mit demselben Druckreservoir 112 verbunden. Dabei wird ähnlich wie in Fig. 13 der Sattdampf 102 jeweils über die Zuleitung 92 durch den Spieß 124 und durch die Strohballen 72 hindurch in den Druckbehälter 84 eingeführt.

Die Weiterbildung von Fig. 14 ist für eine Anlage mit hohem Durchsatz konzipiert, in der die Sattdampfbehandlung sehr effizient durchgeführt werden kann.

### 3.4 Einweichung in Perkolat oder ähnlichem

In dem vierten oben genannten Verfahrensschritt werden die vorbehandelten Ballen in Perkolat eingeweicht, welches eine schwach saure Lösung darstellt. Alternativ können die Ballen jedoch auch in einer schwach alkalischen Lösung, wie beispielsweise Natronlauge, eingeweicht werden. Nach dem Einweichen werden die Ballen auf rund 40°C erwärmt, was beispielsweise dadurch bewerkstelligt werden kann, dass der Stroh-Kanal 46 (s. Fig. 8) durch Abwärme der Gas-Ottomotoren geheizt wird. Durch das Einweichen des Materials nach der Sattdampfbehandlung und vor der anaeroben bakteriellen Fermentation wird eine schwach aerobe Vorhydrolyse initiiert, durch die die anschließende anaerobe bakterielle Fermentation nochmals beschleunigt wird. Bei der Einweichung mit Perkolat sind die anaeroben Bakterien gleich am Ort frischen Materials, was ebenfalls vorteilhaft ist.

Es ist wichtig, festzuhalten, dass mit dem hier beschriebenen Verfahren zum Aufschluss von Stroh erreicht wird, dass das vorbehandelte Material in den Fermentern einen erheblichen Gasertrag bei moderaten Verweilzeiten erbringt, und dies, ohne zusätzliche Enzyme, Pilze oder Hefen zuzufügen. Der vorhandene natürliche Säuregehalt des Strohs (rund 3 bis 4 %) löst auch so die feste Cellulose auf und überführt sie in eine wässrige Lösung (Autohydrolyse). Die biogenen Polymere werden durch die Einwirkung der organischen Säure chemisch und/oder durch Einfluss von Bakterien biochemisch in nieder-molekulare Verbindungen (Monosacharide, Aminosäuren, kurzkettige Peptide, langkettige Fettsäuren, Glyzerin) gespalten. Diese liegen am Ende der Phase in wassergelöster Form vor. Dies geschieht jedoch ohne dass zunächst Enzyme, Bakterien oder Hefen zugegeben werden müssen. Das ligninhaltige Material wird in dieser Ausführungsform allein der Autohydrolyse und der bakteriellen Hydrolyse überlassen.

Der hier im Detail beschriebene Aufschluss mit den genannten vier Verfahrensschritten ist äußerst effektiv und vorteilhaft, jedoch ist es nicht zwingend notwendig, dass sämtliche vier Schritte zur Anwendung kommen, und es können auch einfachere Verfahren mit weniger bzw. nur einer Auswahl der Schritte durchgeführt werden, die immer noch eine Vergärung von ligninhaltigen nachwachsenden Rohstoffen erlauben. Insbesondere kann bereits dann ein brauchbarer Aufschluss des ligninhaltigen Materials erhalten werden, wenn dieses vor der Einbringung in den Fermenter nur mit Festmist und/oder Gülle vermischt wird, weil durch den darin enthaltenen Harnstoff die Ligninstrukturen bereits aufweichen können. Es ist dabei sogar nicht unbedingt nötig, dass das Stroh vor dem Einbringen in den Fermenter mit Gülle oder Festmist vermischt wird, sondern es kann bereits ausreichen, wenn Stroh und Festmist in abwechselnden Lagen im Fermenter übereinander geschichtet werden, gegebenenfalls mit dazwischen liegenden Schichten aus anderen, nicht lignifizierten NawaRos, wobei der Harnstoff der oben liegenden Festmistschichten mit dem Perkolat in die Schicht mit dem ligninhaltigen Material dringt und die flächigen Ligninstrukturen so zumindest teilweise auflöst. Dies stellt einen sehr einfachen Fall eines chemischen Aufschlusses dar.

Ein weiteres sehr effektives Verfahren zum Strohaufschluss stellt die Vermahlung des Strohs dar, wobei der Biogasertrag mit dem Vermahlungsgrad zunimmt.

### 4. Strom- und Wärmeerzeugungsanlage

Die Strom- und Wärmeerzeugungsanlage 22 ist in Fig. 7 vergrößert dargestellt. Sie umfassßt vier Motoraufstellräume 88a bis 88d, die jeweils zum Aufstellen eines Gasottomotors mit angeschlossenem Generator bestimmt sind. Die Gasmotoren nutzen das getrocknete und biologisch entschwefelte, aber ansonsten unveränderte Biogas als Treibstoff. Jeder Motor wird wie oben erwähnt über jeweils einen separaten Gasverdichter versorgt, der das Biogas über Über- und Unterdruckwächter, Gasfilter und Flammenrückschlagsicherungen (nicht gezeigt) aus dem zentralen Gaspeicher 86 zieht und mit einem Druck von 80 bis 200 mbar in die Gasregelstrecke des Motors presst.

Das Biogas wird in den Motoren verbrannt und somit chemische Energie in mechanische Energie und Wärme umgewandelt. Die erforderliche Verbrennungsluft gelangt über ein separates Belüftungssystem 90a bis 90d in den jeweiligen Motoraufstellraum 88a bis 88d. Außerdem ist für jeden Motoraufstellraum 90a bis 90d ein separates Entlüftungssystem 92a bis 92d vorgesehen. Die zugeführte Frischluftmenge ist ca. sechsmal so hoch, wie der Bedarf an Verbrennungsluft.

Die mechanische Energie des Motors wird in den angeschlossenen Mittelspannungs-Generatoren (nicht gezeigt) in elektrischen Strom umgewandelt, der direkt in ein Mittelspannungsnetz eingespeist werden kann. Die anfallende Verbrennungswärme wird über Kühlkreisläufe der Motoren (Kühlung Gas-Luft-Gemisch, Öl-Kühlung, Wasser-Kühlung) (nicht gezeigt) in drei Wärmetauscher abgeführt, die bei jedem Motor in Reihe geschaltet sind. Durch die Motorwärme werden direkt die Fermenter 16, die Perkolatumlauftanks 34, der Frischmassebunker 46, und der Gärrestebunker 60 geheizt. Außerdem kann zusätzliche Wärme zum Heizen eines Verwaltungsgebäudes (nicht gezeigt) verwendet werden. Dies macht jedoch insgesamt lediglich rund 5 % der verfügbaren Wärme aus. Der überwiegende teil der Abwärme wird zur Trocknung von Gärresten in der Dehydrierungs- und Pelletierungseinrichtung 68 verwendet, die unten näher beschrieben wird. Weitere Wärme kann in ein BMKW-eigenes Nahwärmenetz eingespeist werden, über welches beispielsweise benachbarte gewerbliche Betriebe versorgt werden können.

Ferner umfasst die Strom- und Wärmeerzeugungsanlage 22 eine Dockingstation 94. Die Dockingstation ist mit der gesamten Peripherie eines Generatorsets ausgerüstet, insbesondere dem Gasverdichter, der Gasregelstrecke, der Be- und Entlüftungsanlage, Abgaswärmetauschern, Abgasschalldämpfern, Zuleitung Frischöl, Ableitung Altöl und Anschlüssen an das Heizungssystem. Diese Peripherie ist über flexible Anschlüsse mit einem mobilen Generatorset verbindbar, der bei Bedarf herbeigeschafft werden kann.

Falls ein Gasmotor ausfallen sollte, fällt bei dem gezeigten BMKW die Gasverwendungsseite maximal auf 75 % der Leistung ab, und dies auch nur kurzfristig, nämlich bis das mobile Ersatz-Generatorset herbeigeschafft ist. Ein solches Ersatz-Generatorset kann beispielsweise an einer zentralen Stelle in Deutschland auf einem Tieflader für eine Vielzahl von BMKWs bereitgehalten werden und im Bedarfsfall herbeigeschafft und angeschlossen werden.

Die Dockingstation 94 dient jedoch nicht nur für unplanmäßige Ausfälle eines Generatorsets, sondern insbesondere auch für geplante Wartungsmaßnahmen an einem der Generatorsets, so dass die Leistung des BMKW während der Wartungsmaßnahmen nicht heruntergefahren werden muss, weil das ersatzweise Generatorset, welches an die Dockingstation 94 angeschlossen ist, die Arbeit des zu wartenden Generatorsets übernimmt.

Schließlich umfasst die Strom- und Wärmeerzeugungsanlage 22 ein Öllager 96.

### 5. Einrichtung zum Dehydrieren und Pelletieren von Gärresten

Fig. 15 ist ein Blockdiagramm, das die Komponenten der Einrichtung 64 zum Dehydrieren und Pelletieren von Gärresten schematisch darstellt. Die Einrichtung 64 umfasst die Schneckenpresse 70, die oben bereits in Zusammenhang mit Fig. 8 erwähnt wurde. Die Schneckenpresse 70 ist in der Lage, die Gärreste, die sehr nass aus dem Gärrestebunker 60 kommen, bis auf einem Feuchtigkeitsgehalt von 45 bis 55°Gewichts-% zu dehydrieren. Der erzielbare Feuchtigkeitsgehalt wird so eingestellt, dass die verbleibende Restfeuchte von der Abwärme der Gasmotoren ausreichend getrocknet werden kann. Die beim Pressen entstehende flüssige Phase wird als Prozesswasser zurück in den Gärrestebunker 60 geführt. Die feste Phase wird auf das Förderband 66 gegeben, welches in Fig. 8 gezeigt ist und in Fig. 15 durch einen Pfeil symbolisiert ist. Anstelle des Schneckenpresse 70 kann auch ein Dekanter verwendet werden.

Wie weiter in Fig. 15 zu sehen ist, schließt funktional an die Schneckenpresse 70 ein Niedertemperatur-Bandtrockner 142 (sieh auch Fig. 8) an, bei dem während des Trocknungsprozesses aufgrund der niedrigen Temperatur von rund 120 °C keine Dioxine und Furane entstehen. Der Bandtrockner 142 wird ausschließlich mit der Abwärme der Generatorsets beheizt, was den Gesamtprozess sehr effizient macht. Die vom Förderband 66 zugeführten Gärreste fallen in einem Abwurftrichter (nicht gezeigt), aus dem eine Verteileinrichtung sie auf ein darunterliegendes Trocknungsband (nicht gezeigt) auflegt. Die Gärreste bilden auf dem Trocknungsband einen "Teppich", der durch einen Trocknertunnel (nicht gezeigt) gefahren wird. Ein Warmluftstrom durchlüftet den Gärrestteppich und trocknet das Nassgut. Der Trocknungsprozess findet kontinuierlich statt. Während des Trocknungsprozesses wird die Feuchte der Gärreste gemessen, und die Wärmezufuhr wird so geregelt, dass der Wassergehalt der Gärreste bis auf 7 bis 13 Gewichts-% reduziert wird. Ziel der Regelung ist unter anderem, dass der Bandtrockner bei schwankenden Parametern wie Wärmeleistung, Feuchtigkeit der Gärreste, Dehydrierungsbedingungen und Qualität der Gärreste stets den gewünschten niedrigen Feuchtigkeitsgrad der getrockneten Gärreste sicherstellt. Alternativ kann anstelle des Bandtrockners ein Trommeltrockner (nicht gezeigt) mit Kontakttrocknung verwendet werden, der ebenfalls bei ausreichend niedrigen Temperaturen arbeitet.

Der Bandtrockner 142 ist so ausgelegt, dass die anfallende Restwärme möglichst komplett genutzt werden kann, unter besonderer Berücksichtigung von im Sommer entstehenden Abwärmespitzen, die nicht durch den Verkauf z.B. für Heizzwecken nivelliert werden können.

An den Bandtrockner 142 schließt eine pneumatische Förderanlage an, die in der schematischen Darstellung von Fig. 15 durch den Pfeil 144 repräsentiert ist. Da die Gärreste nach der Trocknung im Bandtrockner 142 sehr leicht sind, ist eine pneumatische Förderanlage gegenüber einem Förderband zu bevorzugen, weil während des Transports weniger Material verloren geht und weniger Staub erzeugt wird.

Von der pneumatischen Förderanlage 144 werden die getrockneten Gärreste in eine Hammermühle 146 eingebracht, in der die getrockneten Gärreste zerkleinert werden. Bei der Hammermühle 146 handelt es sich um eine an sich bekannte konventionelle Großraum-Hammermühle auf verbindungssteifen Grundrahmen mit Vibrationsdämpfern, elektrischer Türverriegelung, Lagertemperaturüberwachung und Mahlkammertemperaturüberwachung, wie sie gegenwärtig für die Vermahlung von natürlichen Substraten wie Stroh, Maiskolben, Luzerne, Heu, Papier, Holz, Hanf oder Jute bereits Verwendung findet. Die Hammermühle 146 kann alternativ auch zwischen der Schneckenpresse und dem Bandtrockner angeordnet sein. In diesem Fall findet die Zerkleinerung des Materials bei einem noch höheren Feuchtigkeitsgehalt der Gärreste statt, so dass weniger Staub gebildet wird und die damit verbundene Explosionsgefahr unterbunden wird. Anstelle der Hammermühle 146 kann auch eine Strohmühle verwendet werden.

Die zerkleinerten getrockneten Gärreste werden aus der Hammermühle 146 über eine weitere pneumatische Förderanlage 148 in einen Konditionierer 150 eingebracht. In dem Konditionierer 150 werden die Gärreste mit geeigneten Zusatzstoffen vermengt, durch die die Qualität der schließlich erzeugten Pellets gesteigert wird. Beispielsweise können in dem Konditionierer 150 Getreidemehl und/oder Melasse zugefügt werden, wodurch die Festigkeit der Gärrestepellets erhöht wird, was wiederum die Verbrennung verbessert. Außerdem können die Gärreste im Konditionierer 150 Wasserdampf ausgesetzt werden, wodurch die Masse geschmeidiger wird und sich besser pelletieren lässt. Zusätzlich oder alternativ kann den Gärresten im Konditionierer 150 Branntkalk in geringen Mengen (z.B. 1 bis 2 Gewichts-%) zugefügt werden, um das Ascheerweichungsverhalten der Pellets beim Abbrennen aufgrund des veränderten Verhältnisses von Kalzium zu Kalium in der Asche positiv zu beeinflussen. Schließlich können den Gärresten im Konditionierer 150 Sägemehl oder Holzspäne zugefügt werden, durch die die Qualität der erhaltenen Gärrestepellets als Brennstoff weiter erhöht wird. Ferner können in dem Konditionierer 150 Ölpresskuchen zugefügt werden, die bei der Gewinnung von Öl aus Raps oder Kürbiskernen anfallen und die den Brennwert der Gärrestepellets erhöhen können. Um das Abbrennverhalten der Gärrestepellets zu erhöhen, können außerdem Magnesium, Kalzium und/oder Aluminium beigemischt werden. Ziel der Beimischung ist es, dass die Brennstoffpellets Regelbrennstoffqualität erreichen und/oder für einen Einsatz als Brennstoff gemäß der in den ersten und/oder der vierten Bundesimmissionsschutzverordnung (BImSchV) aufgeführten Bedingungen geeignet sind.

Der Konditionierer 150 ist in der bevorzugten Ausführungsform direkt auf die Pelletierungsvorrichtung 152 aufgesetzt, so dass die im Konditionierer 150 aufbereiteten Gärreste ohne Zwischenspeicher direkt vom Konditionierer in die Pelletierungsvorrichtung 152 eingeführt werden.

Die Pelletierungsvorrichtung 152 wird durch eine an sich bekannte Pelletierpresse gebildet. In der Pelletierpresse 152 wird die aufbereitete Gärmasse in einem Kollergang unter hohem Druck durch Matrizen gepresst, wobei das in den Gärresten enthalten Lignin aufschmilzt, wodurch die Pressmasse zusammengebacken wird. Hinter der Matrize schneidet ein Messer die Pellets zu beispielsweise 15 mm langen Stückchen ab. Alternativ können jedoch auch Brennstoffbriketts hergestellt werden.

Die Pellets werden über eine weitere, ggf. pneumatische Förderanlage 154 in eine Kühleinrichtung 156 eingebracht, in der die Pellets, die sich während der Konditionierung und Pelletierung erhitzt haben, abkühlen können.

Über eine weitere, ggf. pneumatische Förderanlage 158 werden die Pellets in ein Lagersilo 68 transportiert, welches auch in Fig. 8 gezeigt ist, in dem sie bis zu ihrem Abtransport gelagert werden. Die Gärrestepellets sind aufgrund ihres geringen Wassergehalts hydroskopisch, das heißt, sie neigen dazu, Wasser aus der Umgebungsluft aufzunehmen. Daher werden sie im Lagersilo 68 in sehr trockener Atmosphäre gelagert und beispielsweise in einem Tankwagen vom Lagersilo 68 zum Verbraucher transportiert.

Mit der gezeigten Dehydrierungs- und Pelletierungseinrichtung 64 werden aus den Gärresten wertvolle Brennstoffpellets gewonnen. Wenn die Substratmischung für das Vergärungsverfahren vorwiegend aus Stroh und Festmist besteht, werden Gärrestepellets erhalten, deren Brennwert zwischen 5000 und 5400 kWh pro Tonne Trockensubstanz liegt. Dadurch wird ein energetisch und ökonomisch wertvoller Brennstoff geschaffen, zu dessen Erzeugung lediglich Abwärme verwendet wird, die ohnehin anfällt, und ein geringer Bruchteil des ohnehin im Biomassekraftwerk erzeugten Stroms. Da die Gärreste im Fermenter 16 und im Gärrestebunker 60 ausgewaschen werden, hat der erhaltene Brennstoff eine gute Qualität bezüglich Immission. Der Wirkungsgrad des Biomassekraftwerks als Ganzes wird durch die erfindungsgemäße Kombination von Biogaserzeugung durch Vergärung von Stroh und Gärrestepelletierung außerordentlich hoch, wie im folgenden Abschnitt dargelegt wird.

### 6. Energieeffizienz

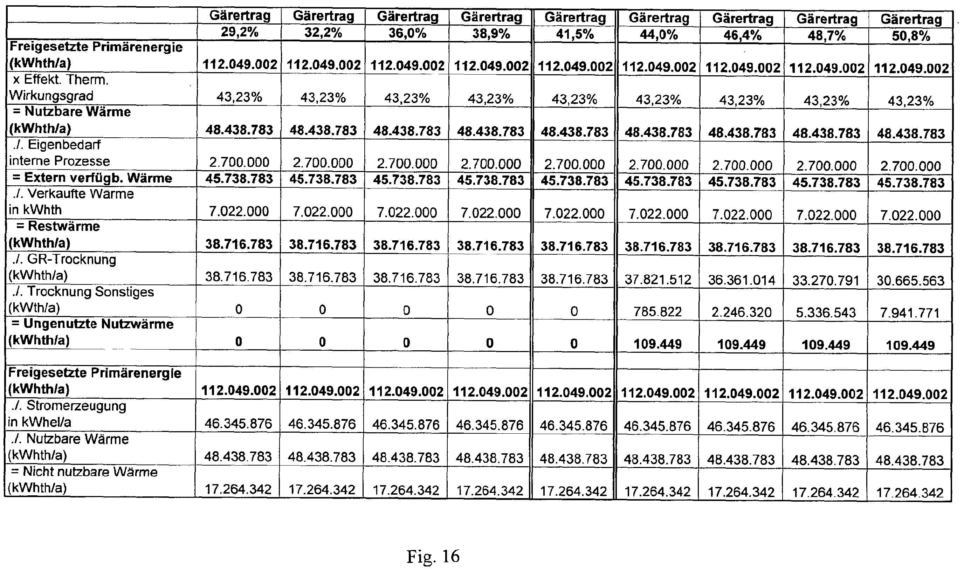

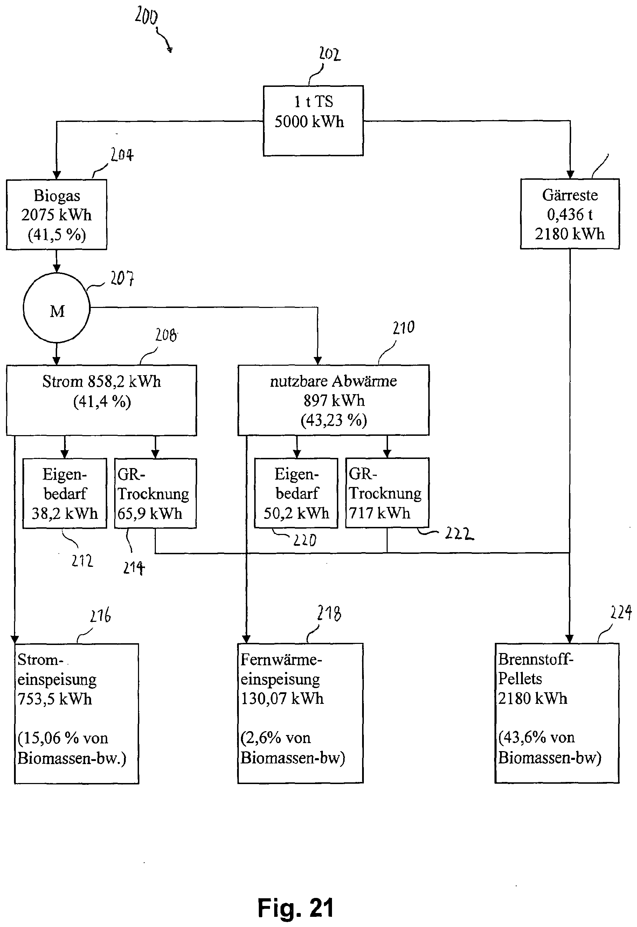

Im Folgenden wird unter Bezugnahme auf die Tabellen von Fig. 16 bis 19 sowie das EnergieFluss-Diagram von Fig. 21 die Energiebilanz des Biomassekraftwerks von Fig. 1 bis 9 diskutiert. In den Tabellen von Fig. 16 bis 19 entsprechen die Spalten unterschiedlichen Betriebsmodi desselben Kraftwerks. Die Betriebsmodi unterscheiden sich in dem relativen Gasertrag, d.h. dem Verhältnis zwischen der im Biogas enthaltenen Energie (in den Tabellen "Freigesetze Primärenergie" genannt) und dem Brennwert des zugefügten Gärsubstrates, wobei der relative Gasertrag in der Tabelle von links nach rechts ansteigt. Dabei entspricht die linkste Spalte einem Betriebsmodus mit einem relativen Gasertrag von 29,2 %, die rechteste Spalte einem Betriebsmodus vom 50,8 %, und die dazwischen liegenden Spalten entsprechen weiteren Betriebsmodi mit sukzessiv ansteigenden relativen Gaserträgen. Die unterschiedlichen Betriebsmodi bzw. relativen Gaserträge ergeben sich durch unterschiedlich aufwendige Verfahren zum Strohaufschluss, von denen vorstehend eine Vielzahl beschrieben wurde, und außerdem durch die Zeitdauer eines Gärzyklusses. Der Betreiber des Biomassekraftwerks hat es also in einem gewissen Rahmen in der Hand, wie hoch er den relativen Gasertrag ansetzen möchte, um zu einem möglichst wirtschaftlichen Betrieb des Kraftwerks zu kommen, wobei höhere Substratsbeschaffungskosten für einen höheren relativen Gasertrag sprechen, jedoch die zusätzlichen Kosten für einen dafür nötigen erhöhten Strohaufschluss gegenzurechnen sind.

In allen Betriebsmodi wird dieselbe Menge an Biogas anvisiert, die so bemessen ist, dass die Gasmotoren ständig unter Volllast laufen können. Das in Fig. 1 bis 9 gezeigte Biomassekraftwerk, auf welches sich die hier beschriebene Energiebilanz bezieht, ist ein 5,6 MW Kraftwerk. Die in den Tabellen aufgeführten Energiemengen, Gärsubstratmengen und Gärrestemengen beziehen sich jeweils auf ein Jahr.

Konkret zeigt die Tabelle von Fig. 16 den benötigten Input an Gärsubstrat (gemessen in Tonnen Trockensubstanz), die für den benötigten absoluten Gasertrag ("Freigesetzte Primärenergie") benötigt wird, sowie den Wert der erzeugbaren Ströme, von dem der Strom für den Eigenbedarf des Biomassekraftwerks und für die Gärresttrocknung abgezogen werden muss. Obwohl nämlich in allen Betriebsmodi, d.h. in allen Spalten der Tabellen, dieselbe Gesamtmenge an Strom erzeugt wird, wird bei Betriebsmodi mit höherem Substratdurchsatz (d.h. geringerem relativen Gasertrag) mehr Strom für den Betrieb des Kraftwerks benötigt, so dass der effektiv einspeisbare Strom sich zwischen den Betriebsmodi leicht unterscheidet.

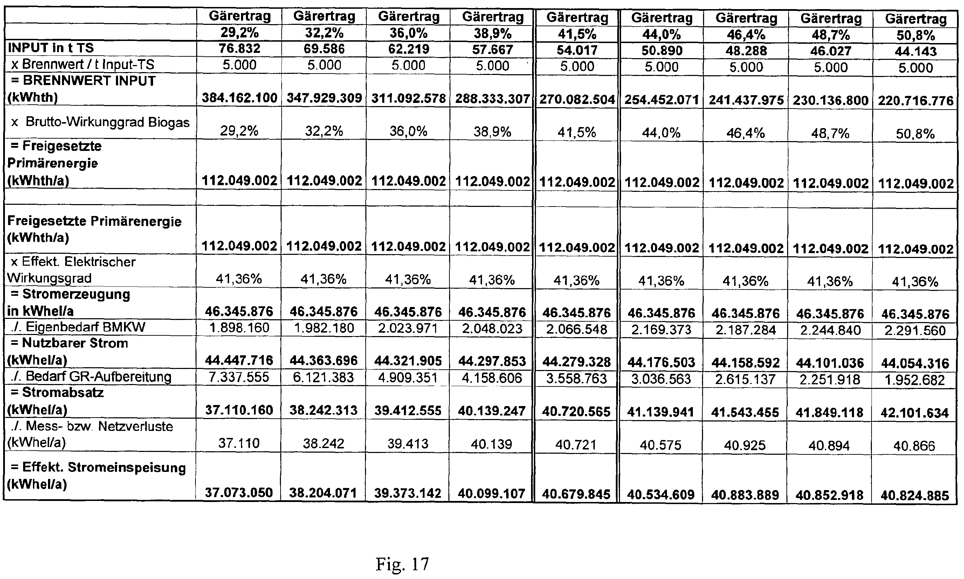

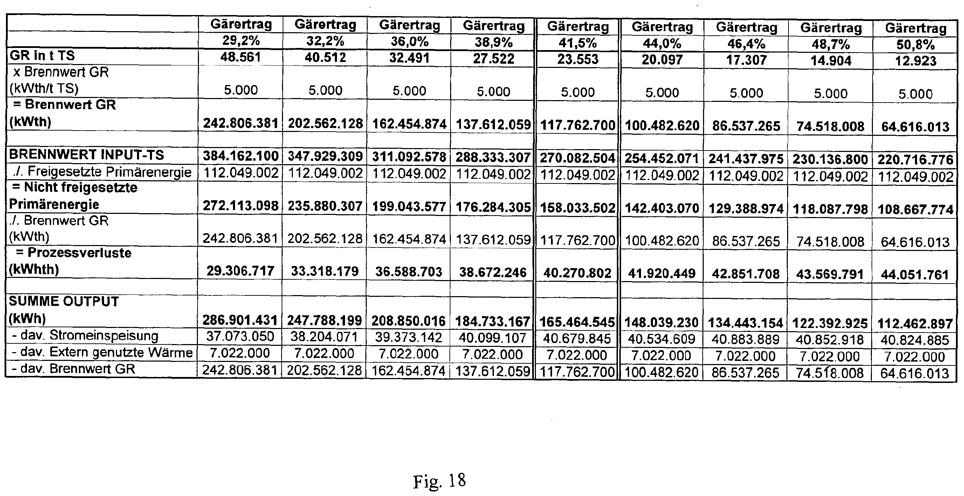

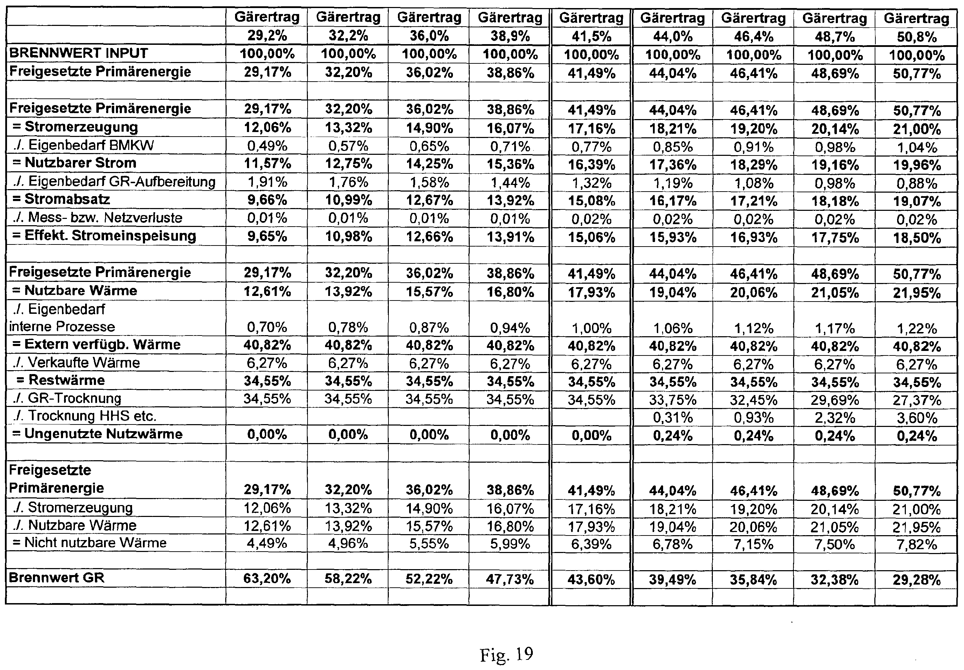

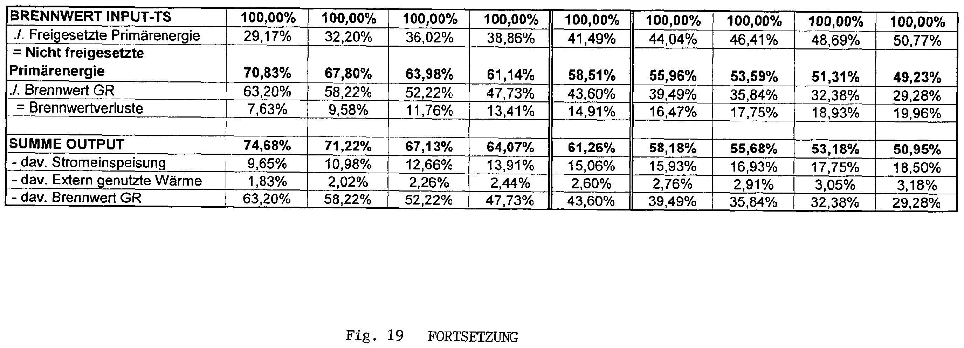

Die Tabelle von Fig. 17 fasst den Wärmefluss zusammen, und die Tabelle von Fig. 18 bezieht sich auf die Energie der Gärrestepellets, beides in Absolutzahlen bemessen auf ein Jahr. Schließlich fasst die Tabelle von Fig. 19 die Werte von Tabellen 16, 17 und 18 noch einmal zusammen, wobei die jeweiligen Energien als prozentuale Bruchteile des gesamten Brennwertes der Trockensubstanz zugeführten Frischmasse ausgedrückt sind.

Um die Energiebilanztabellen von Fig. 16 bis 19 und ihre technische Aussagekraft näher zu erläutern, soll im Folgenden beispielhaft die jeweils fünfte Spalte herausgegriffen werden. Zu diesem Zwecke ist die Spalte 5 in den Tabellen von Fig. 16 bis 19 optisch hervorgehoben. Die darin enthaltenen Zahlen werden in Zusammenhang mit dem Energie-Flussdiagram 200 von Fig. 21 näher diskutiert.

Wie der Spalte 5 der Tabelle von Fig. 16 zu entnehmen ist, beträgt der relative Gasertrag in dem zugehörigen Betriebsmodus 41,5 %. Um auf eine benötigte Biogas-Energiemenge von 112.049.002 kWh im Jahr zu kommen, werden bei diesem relativen Gasertrag und einem Brennwert von etwa 5.000 kWh pro Tonne Trockensubstanz 54.107 Tonnen Trockensubstanz benötigt. Fig. 21 zeigt den Fluss der mit der Trockensubstanz zugeführten Energie im Kraftwerk von Fig. 1 bis 9 und im Betriebsmodus von Spalte 5, jedoch bezogen auf eine Tonne eingegebener Trockensubstanz. In dem Energie-Flussdiagram von Fig. 21 repräsentiert der Kasten 202 eine t TS Biomasse mit einer Feuerwärmeleistung von 5000 kWh.

Im Zuge der Vergärung in dem Biomassekraftwerk von Fig. 1 bis 9 wird aus dieser einen Tonne Biomasse Biogas mit einem Brennwert von 2.075 kWh erzeugt, entsprechend dem relativen Gasertrag von 41,5 % (siehe Tabelle 16), und dieses Biogas wird durch den Kasten 204 repräsentiert. Wie der Tabelle von Fig. 18 zu entnehmen ist, werden pro Tonne Frischmasse 0,436 Tonnen Gärreste erzeugt, die ebenfalls eine Feuerwärmeleistung von rund 5.000 kWh pro Tonne aufweisen und somit einen Brennwert von 2.180 kWh haben (Kasten 206).

Das Biogas von Kasten 204 wird in dem Generatorset 206, welches aus einem Gasmotor und einen Stromgenerator besteht, verbrannt. Die verwendeten Generatorsets 206 haben einen effektiven elektrischen Wirkungsgrad von 41,36 % (siehe Tabelle von Fig. 16), so dass 858,2 kWh Strom erzeugt werden (Kästchen 208). Wie die Tabelle von Fig. 17 zu entnehmen ist, werden 43,23 % der Primärenergie des Biogases im Generatorset im Abwärme umgewandelt, so dass 897 kWh an nutzbarer Abwärme entstehen (Kästchen 210).

Wie der Tabelle von Fig. 16 zu entnehmen ist, werden von den 858,2 kWh Strom 38,2 kWh (4,45 %) für den Betrieb des Biomassekraftwerks benötigt (Kästchen 212). Ferner werden 65,9 kWh (7,68 %) für die Gärrestetrocknung benötigt (Kästchen 214). Die verbleibenden 753,5 kWh (87,8 %) können dann in einem Stromnetz eingespeist werden (Kästchen 216). Dies entspricht 15,06 % des Brennwerts der ursprünglich eingesetzten Biomasse (siehe Tabelle von Fig. 19).

Von den 897 kWh Abwärme (Kästchen 210) werden in dem hier gezeigten Ausführungsbeispiel 130,07 kWh als Fernwärme verkauft (Kästchen 218), dies entspricht 14,5 % der anfallenden Abwärme (siehe Tabelle von Fig. 17). Ferner werden 50,23 kWh (5,6 %) der Abwärme für den Betrieb des Biomassekraftwerks benötigt, beispielsweise zum Erwärmen der Fermenter 16 und des Gärrestebunkers 60 (Kästchen 220). Wie weiterhin aus der Tabelle von Fig. 17 zu entnehmen ist, werden der größte Teil der Abwärme, nämlich 716,97 kWh bzw. 80 % der gesamten Abwärme für die Gärrestetrocknung verwendet (Kästchen 222).

Das Verhältnis von verkaufter Fernwärme zu Wärme, die für die Gärrestetrocknung eingesetzt wird, erscheint auf den ersten Blick nicht attraktiv. Tatsächlich wäre es auch möglich, einen geringeren Teil der Abwärme für die Gärrestetrocknung zu nehmen (und dafür die mechanische Dehydrierung etwas intensiver zu betreiben). Das Ausführungsbeispiel ist jedoch insofern realistisch, als in vielen Fällen kein beliebig großer Bedarf an Fernwärme besteht. Der Betreiber des Biomassekraftwerks hat sich dem tatsächlich vorhandenen Bedarf an Fernwärme anzupassen. Tatsächlich gibt es eine Vielzahl an sich sehr attraktive Standorte für Biomassekraftwerke, an denen überhaupt kein Bedarf an Fernwärme besteht. In diesen Fällen stellt die Gärrestetrocknung eine sehr attraktive Weise dar, die Abwärme dennoch sinnvoll zu nutzen. In den hier gezeigten Betriebsmodi wurde in Übereinstimmung mit einem konkret geplanten Projekt angenommen, dass ein Abnehmer für Fernwärme 7.022.000 kWh pro Jahr abnehmen möchte (siehe Tabelle von Fig. 17). Die übrige nicht benötigte Abwärme wird vollständig für die Gärrestetrocknung verwendet, d.h. die Gärreste werden mechanisch nur so weit dehydriert, dass die verbleibende Abwärme noch für ihre Trocknung ausreicht. Es kann also im Gegenzug Strom für den Betrieb der Schneckenpresse 70 eingespart werden.

Schließlich werden die Gärreste von Kästchen 206 unter Verwendung von Abwärme (Kästchen 222) und unter Einsatz von Strom (Kästchen 214) zu Brennstoffpellets verarbeitet (Kästchen 224), die einen Brennwert von 2.180 kWh aufweisen und somit 43,6 % des Brennwerts der ursprünglichen Biomasse als hochwertigen Brennstoff bereitstellen.

Zusammenfassend werden in dem Betriebsmodus von Spalte 5 von dem Brennwert der eingesetzten Biomasse 15,06 % als Strom ins Netz eingespeist, 2,6 % als Fernwärme verfügbar gemacht und 43,6 % als hochwertiger Regelbrennstoff bereitgestellt. Dies bedeutet, dass 61,36 % des Brennwerts der eingesetzten Biomasse nutzbar gemacht werden. Dies stellt eine erhebliche Steigerung gegenüber bekannten Biomassekraftwerken dar, deren theoretische Netto-Wirkungsgrade bei 23 bis 42 % liegen und deren Netto-Wirkungsgrade in der Praxis im Bereich von 10 bis 20 % liegen.

Obwohl nur ein Betriebsmodus, nämlich der Betriebsmodus von Spalte 5 im Detail diskutiert wurde, zeigen die Tabellen von Fig. 16 bis 19, dass das Verfahren und das Biomassekraftwerk auch bei deutlich niedrigeren und deutlich höheren relativen Gaserträgen insgesamt sehr wirtschaftlich arbeiten. Dies ist ein wesentlicher Vorteil der Erfindung, weil es dem Betreiber die Möglichkeit gibt, das Biomassekraftwerk in Abhängigkeit von den Marktpreisen für Biomasse (insbesondere Stroh), eingespeistem Strom, eingespeister Wärme und für Gärrestepellets als Brennstoff so zu betreiben, dass es für ihn wirtschaftlich ist. Die Wirtschaftlichkeit ist zwar grundsätzlich ein ökonomisches Problem, für das das erfindungsgemäße Biomassekraftwerk und das erfindungsgemäße Verfahren jedoch eine technische Lösung anbietet. Obgleich in den Zeichnungen und der vorhergehenden Beschreibung ein bevorzugtes Ausführungsbeispiel aufgezeigt und detailliert beschrieben ist, sollte dies als rein beispielhaft und die Erfindung nicht einschränkend angesehen werden. Es wird darauf hingewiesen, dass nur das bevorzugte Ausführungsbeispiel dargestellt und beschrieben ist und sämtliche Veränderungen und Modifizierungen, die derzeitig und künftig im Schutzumfang der Erfindung liegen, geschützt werden sollen.

### Bezugszeichenliste

- 10: Biomassekraftwerk
- 12: Grundabschnitt
- 14: Ausbauabschnitt
- 16: Fermenter
- 18: Fermentervorplatz
- 20: Fermentertor
- 22: Strom- und Wärmeerzeugungsanlage
- 24: Anliefer- und Verladebereich
- 26: Fermentervorplatz-Hallenabschnitt
- 28: Anliefer- und Verladebereichshallenabschnitt
- 30: Technikbrücke
- 31: Abgas-Abkühlraum
- 32: Folien-Gasspeicher
- 34: Perkolatumlauftank
- 36: Südlicher Technikraum
- 38: Nördlicher Technikraum
- 40: Lichtbänder
- 42: Anlieferbunker für Frischmasse
- 44: Förderband
- 46: Frischmassebunker
- 48: Bereitstellungsraum
- 50: Ballen-Anlieferungsraum
- 52: Aufschlußbereich
- 54: Zwischenlager
- 56: Rollenförderer
- 58: Strohkanal
- 60: Gärrestebunker
- 62: Schütttrog für Gärreste
- 64: Einrichtung zur Dehydrierung und Pelletierung
- 66: Förderband für Gärreste
- 68: Lagersilo für Gärrestepellets
- 70: Schneckenpresse
- 72: Strohballen
- 74: Unterseite des Strohballens 72
- 76: Oberseite des Strohballens 72
- 78: erster Satz von Löchern
- 80: zweiter Satz von Löchern
- 82: Einrichtung zur Sattdampfbehandlung
- 83: Deckel
- 84: Druckbehälter
- 85: Scharnier
- 86: Zentraler Gasverteilspeicher
- 87: Zufuhreinrichtung
- 88: Motoraufstellraum
- 89: Behälter
- 90: Zuluft Motoraufstellräume
- 91: Schließmechanismus
- 92: Entlüftung Motoraufstellräume
- 93: Zufuhrleitung
- 94: Docking Station
- 95: Zuführungsventil
- 96: Lager
- 97: Auslaßleitung
- 98: Auslaß-Ventil
- 100: Kompressor
- 102: Sattdampf
- 103: Austretender Sattdampf
- 104: Einrichtung zur Sattdampfbehandlung
- 106: ligninhaltiger nachwachsender Rohstoff
- 108: druckfester Schieber
- 110: Trichter
- 112: Dampfreservoir
- 114: Heizung
- 116: Förderband
- 117: Slurry
- 118: Rohrverbindung
- 120: Leitung zum PUT
- 122: Einrichtung zur Sattdampfbehandlung
- 124: Spieß
- 126: innerer Hohlraum
- 128: Öffnung im Spieß 124
- 130: Einrichtung zur Sattdampfbehandlung
- 132: Kran
- 134: Laufkatze
- 136: Gestell
- 138: Greifer für oberen Ballen
- 140: Greifer für unteren Ballen
- 142: Bandtrockner
- 144: Pneumatische Förderanlage
- 146: Hammermühle
- 148: Pneumatische Förderanlage
- 150: Konditionierer
- 152: Pelletierungsvorrichtung
- 154: Pneumatische Förderanlage
- 156: Kühleinrichtung
- 158: Pneumatische Förderanlage
- 200: Energieflussdiagramm
- 202-224: Energieanteile im Energieflussdiagramm 200

## Patentansprüche

1. Biogasanlage (10) zur Erzeugung von Biogas aus ligninhaltigen nachwachsenden Rohstoffen, die zumindest teilweise aus Stroh bestehen, umfassend:
eine Mehrzahl von garagenförmigen Fermentern (16) für eine anaerobe bakterielle Vergärung der ligninhaltigen nachwachsenden Rohstoffe, die zumindest teilweise aus Stroh bestehen, als Gärsubstrat nach dem Feststoffvergärungsverfahren auf mesophilem Temperaturniveau, und eine Einrichtung (64) zur Gewinnung von Brennstoffpellets und/oder Brennstoffbriketts durch Dehydrieren und Pelletieren der Gärreste.

2. Biogasanlage (10) nach Anspruch 1, mit einer Einrichtung (82, 104, 122, 130) zum chemischen, mechanischen und/oder thermischen Aufschluss von nachwachsenden ligninhaltigen Rohstoffen, die zumindest teilweise aus Stroh bestehen,
wobei die Einrichtung zum Aufschluss von Stroh eine Einrichtung zur mechanischen Zerkleinerung von Stroh, insbesondere eine Hammermühle oder Strohmühle, und/oder eine Einrichtung zur Sattdampfbehandlung umfasst,
wobei die Einrichtung (82, 104, 122, 130) zur Sattdampfbehandlung vorzugsweise einen Druckbehälter (84) und Mittel (112, 114) umfasst, die geeignet sind, im Druckbehälter (84) einen Wasserdampf zu erzeugen, mit einem Druck, der zwischen 20 und 30 bar liegt und einer Temperatur, die zwischen 180°C und 250°C liegt und/oder einen Behälter zum Einweichen des Strohs in Wasser, einer Wasser-Laugen-Lösung, einer Wasser-Säure-Lösung, Perkolat oder Gülle umfasst.

3. Biogasanlage (10) nach Anspruch 1 oder 2, mit einer Einrichtung (60) zum Auswaschen oder Eluieren der Gärreste, und/oder
mit mindestens einem Gasmotor und mindestens einem Stromgenerator, der durch den Gasmotor betreibbar ist.

4. Biogasanlage (10) nach einem der vorhergehenden Ansprüche, bei der die Einrichtung (64) zur Gewinnung von Brennstoffpellets und/oder Brennstoffbriketts eine Einrichtung zum mechanischen Dehydrieren von Gärresten, insbesondere eine Schneckenpresse (70) oder einen Dekanter umfasst und/oder
eine Trocknungseinrichtung, insbesondere einen Bandtrockner (142) oder einen Trommeltrockner mit Kontakttrocknung umfasst, wobei
die Trocknungseinrichtung (142) vorzugsweise mit Abwärme von mindestens einem Gasmotor der Biogasanlage (10) betrieben wird und bei der die Temperatur der Trocknungseinrichtung vorzugsweise 180 °C, vorzugsweise 140 °C nicht übersteigt.

5. Biogasanlage (10) nach einem der vorhergehenden Ansprüche, bei der die Einrichtung (64) zur Gewinnung von Brennstoffpellets und/oder Bennstoffbriketts eine Einrichtung zum Zerkleinern der zumindest teilweise dehydrierten und gegebenenfalls getrockneten Gärreste, insbesondere eine Hammermühle (146) oder eine Strohmühle umfasst, und/oder eine Konditioniereinrichtung (150) umfasst, die geeignet ist, den zerkleinerten und zumindest teilweise dehydrierten und/oder getrockneten Gärresten eine oder mehrere der folgenden Zugaben beizumischen: Getreidemehl, Melasse, Branntkalk, Dampf, Holzspäne, Sägemehl, Ölpresskuchen, Calzium, Magnesium, Aluminium, Stickstoff-bindende Mittel, insbesondere Stickstoff-reduzierende Reduktionsmittel, Kalkhydrat, und/oder eine Pelletierpresse (152) umfasst, die geeignet ist, die zerkleinerten und dehydrierten Gärreste zu Pellets oder Briketts zu pressen, wobei
vorzugsweise zumindest einige der Komponenten der Einrichtung (64) zum Dehydrieren und Pelletieren von Gärresten durch eine pneumatische Förderanlage (144, 148, 154, 158) verbunden sind, und/oder
mit einem Anliefer- und Verladebereich (24), wobei
die Einrichtung zum chemischen, mechanischen und/oder thermischen Aufschluss von Stroh vorzugsweise in dem Anliefer- und Verladebereich (24) untergebracht ist, und/oder bei der der Anliefer- und Verladebereich (24) vorzugsweise stationäre Fördertechnik (44, 46, 56) umfasst, die geeignet ist, Biomasse aus dem Anliefer- und Verladebereich (24) zu einem Fermentervorplatz (28) zu fördern, von dem aus eine Mehrzahl von Fermentern (16) des Garagentyps zugänglich sind, und/oder
bei der der Anliefer- und Verladebereich (24) mindestens einen eingehausten Anlieferbunker (42) für Frischmasse umfasst.

6. Biogasanlage (10) nach Anspruch 5, mit ersten Fördermitteln (44), die geeignet sind, Frischmasse aus dem mindestens einen Anlieferbunker (42) für Frischmasse zu einem Frischmassebunker (46) zu befördern, wobei
die ersten Fördermittel vorzugsweise ein Förderband (44) umfassen, auf dem Frischmasse aus verschiedenen Anlieferbunkem (42) zum Frischmassebunker (46) beförderbar ist, optional mit zweiten Fördermitteln, insbesondere einem Schubschild, welche geeignet sind, die Frischmasse durch den Frischmassebunker (46) in Richtung auf den Fermentervorplatz (18) zu befördern, wobei
innerhalb des Anliefer- und Verladebereichs (24) vorzugsweise eine Entladungsstelle (50) mit Kran und ein Zwischenlager für Ballenmaterial, insbesondere für Stroh vorgegeben ist, und wobei vorzugsweise
dritte Fördermittel, insbesondere Rollenförderer oder Schubförderer, vorgesehen sind, die geeignet sind, einzelne Ballen oder Pakete von Ballen entlang eines Ballenkanals (58) zum Fermentervorplatz (18) zu befördern.

7. Biogasanlage (10) nach einem der vorhergehenden Ansprüche, mit einem Gärrestebunker (60), der vom Fermentervorplatz (18) aus zum Einbringen von Gärresten zugänglich ist, wobei der Gärrestebunker (60) vorzugsweise stationäre Fördermittel, insbesondere Förderschnecken umfasst, die geeignet sind, Gärreste durch den Gärrestebunker (60) hindurch zu transportieren, wobei der Gärrestebunker (60) vorzugsweise an das Biogassystem angeschlossen ist.

8. Verfahren zur Erzeugung von Biogas aus nachwachsenden ligninhaltigen Rohstoffen, die zumindest teilweise aus Stroh bestehen, in einer Biogasanlage (10) gemäß einem der Ansprüche 1-7, wobei das Verfahren die folgenden Schritte aufweist:
Einbringen der ligninhaltigen nachwachsenden Rohstoffe, die zumindest teilweise aus Stroh bestehen, als Gärsubstrat in einen garagenförmigen Fermenter, der für eine Feststoffvergärung geeignet ist,
Erzeugen von mesophilen Bedingungen im Fermenter (16), die eine anaerobe bakterielle Vergärung erlauben,
Entfernen von Gärresten aus dem Fermenter, und
Gewinnen von Brennstoffpellets oder Brennstoffbriketts durch Dehydrieren und Pelletieren der Gärreste.

9. Verfahren nach Anspruch 8, bei dem das Stroh vorbehandelt wird, um einen chemischen, thermischen und/oder mechanischen Aufschluss desselben zu bewirken, und/oder
die Trockensubstanz des zur Vergärung verwendeten Substrats vorzugsweise zu mindestens 30 Gewichts-%, vorzugsweise mindestens 50 Gewichts-% aus Stroh besteht, oder
bei dem die Trockensubstanz des zur Vergärung verwendeten Substrats vorzugsweise 30 Gewichts-% bis 90 Gewichts-% reines Stroh, 10 Gewichts-% bis 70 Gewichts-% Festmist und 0 Gewichts-% bis 30 Gewichts-% weitere Substrate enthält.

10. Verfahren nach einem der Ansprüche 8 oder 9, bei dem für die Verbrennung schädliche Stoffe, insbesondere Chlor, Stickstoff, Schwefel, Kaliumchlorid und/oder Silikate aus den Gärresten nach ihrer Entfernung aus dem Fermenter ausgewaschen oder eluiert werden, wobei
die Auswaschung der Gärreste vorzugsweise im Gärrestebunker (60) vorgenommen wird, und/oder
die Gärreste nach dem Auswaschen mechanisch dehydriert werden, insbesondere un ter Verwendung einer Schneckenpresse (70) oder eines Dekanters, wobei die entstehende flüssige Phase als Prozesswasser zurück in den Gärrestebunker (60) geführt wird und/oder
die Gärreste in einer Trocknungseinrichtung, insbesondere einem Bandtrockner (142) oder einem Trommeltrockner mit Kontakttrocknung vorzugsweise mit Abwärme von mindestens einem Gasmotor der Biogasanlage (10) getrocknet werden, und/oderdie zumindest teilweise dehydrierten und gegebenenfalls getrockneten Gärreste mechanisch zerkleinert werden, insbesondere in einer Hammermühle (146) oder Strohmühle, und/oder
den zerkleinerten und zumindest teilweise dehydrierten und/oder getrockneten Gärresten eine oder mehrere der folgenden Zugaben beigemischt werden: Getreidemehl, Melasse, Branntkalk, Dampf, Holzspäne, Sägemehl, Ölpresskuchen, Calzium, Magnesium, Aluminium, Stickstoff-bindende Mittel, insbesondere Stickstoff-reduzierende Reduktionsmittel, Kalkhydrat, und/oder
die zerkleinerten und dehydrierten Gärreste zu Pellets oder Briketts gepresst werden.

11. Verfahren nach einem der Ansprüche 9 bis 10, bei dem die Vorbehandlung zum thermischen Aufschluss eine Sattdampfbehandlung umfasst, wobei
die Sattdampfbehandlung vorzugsweise so durchgeführt wird, dass sie Ligninstrukturen des Strohs aufweicht, die äußere Struktur des Rohstoffs insgesamt aber im Wesentlichen bestehen bleibt, und/oder
die Sattdampfbehandlung bei einer Temperatur von zwischen 160°C und 240°C und einem Druck von zwischen 20 und 30 bar für weniger als 20 Minuten durchgeführt wird,
und/oder
der Behandlungsdruck am Ende der Sattdampfbehandlung innerhalb von fünf Sekunden um mindestens 80% gesenkt wird, und/oder
das Stroh vor der Sattdampfbehandlung eingeweicht wird, und/oder
das Stroh nach der Sattdampfbehandlung in einer sauren Lösung, insbesondere Perkolat, in einer alkalischen Lösung oder in Gülle eingeweicht wird, und/oder
das Stroh vor der weiteren Verwendung, insbesondere vor der Sattdampfbehandlung mechanisch zerkleinert wird, und/oder
das Stroh in Ballenform bereitgestellt wird, wobei
die Ballen (72) vorzugsweise eine Dichte von wenigstens 200 kg/m³, vorzugsweise von wenigstens 208 kg/m³ aufweisen, und/oder
die Ballen (72) als unterste Schicht in einen Fermenter (16) geschichtet werden, und/oder die Ballen aufgelöst werden, nachdem sie in den Fermenter (16) geschichtet wurden.

12. Verfahren nach einem der Ansprüche 9 bis 11, bei dem die chemische Vorbehandlung eine Vermischung des Strohs mit Festmist, Gülle, Perkolat und/oder perkolatenthaltender Gärmasse umfasst, und/oder
bei dem die chemische Vorbehandlung das Einweichen von Stroh in einer Wasser- SäureLösung, einer Wasser-Laugen-Lösung, Perkolat oder Gülle umfasst, und/oder das Stroh vor dem Einsatz als Gärsubstrat zerfasert wird.

13. Verfahren nach einem der Ansprüche 9 bis 12, bei dem die Vorbehandlung des Strohs zum mechanischen Aufschluss das Häckseln und/oder Schneiden zumindest eines Anteils desselben umfasst, wobei
das Stroh vorzugsweise auf eine Partikellänge von weniger als 5 cm, vorzugsweise weniger als 2 cm geschnitten und/oder gehäckselt wird, und/oder
bei dem zwischen 10 % und 70 % des Strohs geschnitten und/oder gehäckselt wird, und/oder
bei dem die Vorbehandlung des Strohs zum mechanischen Aufschluss das Vermahlen zumindest eines Anteils desselben umfasst, wobei
zwischen 5 % und 100 %, vorzugsweise zwischen 20 % und 65% des Strohs vermahlen werden, und/oder
der Vermahlungsgrad des Strohs einem Sieblochdurchmesser von höchstens 8 mm, vorzugsweise einen Sieblochdurchmesser von höchstens 1 mm entspricht, und/oder dem Stroh zwischen der Vorbehandlung und dem Einbringen in den Fermenter zur anaeroben Fermentation keine zusätzlichen Säuren, Enzyme, Pilze oder Hefen von außen zugeführt werden.

14. Verfahren nach einem der Ansprüche 8 bis 13, bei dem bei einer Auffrischung der Gärmasse ein Teil der aus dem Fermenter (16) entnommenen Gärmasse mit einer Presse, insbesondere mit einer Schneckenpresse, dehydriert und als Animpfmasse mit neuer Frischmasse vermischt wird und anschließend als aufgefrischte Gärmasse wieder in den Fermenter (16) gegeben wird, und/oder die Gärreste, die dem Fermenter (16) entnommen werden, in einem Gärrestebunker (60) nachvergoren werden, wobei die Temperatur im Gärrestebunker (60) 50 bis 58°C beträgt, und der Gärrestebunker (60) an das Biogassystem angeschlossen ist, wobei vorzugsweise Enzyme in den Gärrestebunker (60) eingeführt werden, die den Wirkungsgrad der Nachvergärung steigern.

15. Verfahren nach einem der Ansprüche 8 bis 14, bei dem den ausgewaschenen oder eluierten, dehydrierten und zerkleinerten Gärresten vor deren Pelletierung soviel Melasse, Branntkalk, Kalkhydrat, Holzspäne, Sägemehl, Calzium, Magnesium und/oder Aluminium beigemischt wird, dass die Pellets oder Briketts folgende Brennstoffqualität erreichen:
als Bestandteile im Abgas bei einem Bezugssauerstoffvolumen von 13%: 4 g CO/m³, 150 mg Staub/Nm³,
vorzugsweise als Bestandteile im Abgas bei einem Bezugssauerstoffvolumen von 11%: 0,25 g CO/Nm³, 50 g Staub/Nm³, 50 mg Gesamt-C/Nm³, 500 mg NOₓ,/Nm³, 2,0 g SO₂/Nm³, und
insbesondere vorzugsweise als Bestandteile im Abgas bei einem Bezugssauerstoffvolumen von 11%: 0,25 g CO/Nm³, 20 g Staub/Nm³, 50 mg Gesamt-C/Nm³, 400 mg NOₓ/Nm³, 2,0 g SO₂/Nm³, und/oder die Gärreste vor der Pelletierung zerfasert und/oder entstaubt werden.

## Claims

1. A biogas plant (10) for producing biogas from lignin-containing renewable resources, which at least in part consist of straw, comprising:
a plurality of garage-shaped fermenters (16) for an anaerobic bacterial fermentation of the lignin-containing renewable resources, which at least in part consist of straw, as a fermentation substrate according to the solid fermentation method at a mesophilic temperature level, and a device (64) for producing fuel pellets and/or fuel briquettes by dehydrating and pelleting the fermentation residues.

2. The biogas plant (10) according to claim 1, comprising a device (82, 104, 122, 130) for the chemical, mechanical and/or thermal decomposition of renewable lignin-containing resources, which at least in part consist of straw,
wherein the device for decomposing straw comprises a device for mechanically comminuting straw, in particular a hammer mill or straw mill, and/or a device for the saturated steam treatment,
wherein the device (82, 104, 122, 130) for the saturated steam treatment preferably comprises a pressure container (84) and means (112, 114), which are suitable to produce a steam in the pressure vessel (84) at a pressure which is between 20 and 30 bar and a temperature which is between 180°C and 250°C, and/or a container for soaking the straw in water, a water-lye solution, a water-acid solution, percolate or liquid manure.

3. The biogas plant (10) according to claim 1 or 2, comprising a device (60) for washing out or eluting the fermentation residues and/or comprising at least one gas engine and at least one power generator which can be operated by the gas engine.

4. The biogas plant (10) according to any of the preceding claims, in which the device (64) for producing fuel pellets and/or fuel briquettes comprises a device for mechanically dehydrating fermentation residues, in particular a screw press (70) or a decanter, and/or
a drying device, in particular a belt drier (142) or a drum drier with contact drying, wherein
the drying device (142) is preferably operated with waste heat from at least one gas engine of the biogas plant (10) and wherein the temperature of the drying device does preferably not exceed 180°C and preferably not exceed 140°C.

5. The biogas plant (10) according to any of the present claims, in which the device (64) for producing fuel pellets and/or fuel briquettes comprises a device for comminuting the at least in part dehydrated and optionally dried fermentation residues, in particular a hammer mill (146) or a straw mill, and/or comprises a conditioning device (150), which is suitable to mix the comminuted and at least in part dehydrated and/or dried fermentation residues with one or more of the following additives:
cereal flour, molasses, caustic lime, steam, chipped wood, sawdust, oil press cake, calcium, magnesium, aluminum, nitrogen-binding agents, in particular, nitrogen-reducing reductants, hydrated lime, and/or comprises a pelleting press (152), which is suitable to press the comminuted and dehydrated fermentation residues into pellets or briquettes, wherein preferably at least some of the components of the device (64) are connected for dehydrating and pelleting fermentation residues by a pneumatic conveyor system (144, 148, 154, 158), and/or
comprising a delivery and loading area (24), wherein
the device is preferably accommodated in the delivery and loading area (24) for the chemical, mechanical and/or thermal decomposition of straw, and/or in which the delivery and loading area (24) preferably comprises stationary conveyor technique (44, 46, 56), which is suitable to convey biomass from the delivery and loading area (24) to a fermenter forecourt (28) from where a plurality of garage-type fermenters (16) can be accessed, and/or
in which the delivery and loading area (24) comprises at least one enclosed delivery bunker (42) for fresh mass.

6. The biogas plant (10) according to claim 5, with first means of conveyance (44), which are suitable to convey fresh mass from the at least one delivery bunker (42) for fresh mass to a fresh mass bunker (46), wherein
the first means of conveyance preferably comprise a conveyor belt (44), on which fresh mass from various delivery bunkers (42) can be conveyed to the fresh mass bunker (46),
optionally with second means of conveyance, in particular a thrust plate, which are suitable to convey the fresh mass through the fresh mass bunker (46) towards the fermenter forecourt (18), wherein
an unloading point (50) with crane and a temporary storage for bale material, in particular for straw, is preferably given within the delivery and loading area (24), and wherein
third means of conveyance, in particular roller conveyors or pushing conveyors, are preferably provided, which are suitable to convey individual bales or packages of bales along a bale channel (58) to the fermenter forecourt (18).

7. The biogas plant (10) according to any of the preceding claims, comprising a fermentation residue bunker (60), which can be accessed from the fermenter forecourt (18) to introduce fermentation residues, wherein the fermentation residue bunker (60) preferably comprises stationary means of conveyance, in particular screw conveyors which are suitable to transport fermentation residues through the fermentation residue bunker (60), wherein the fermentation residue bunker (60) is preferably connected to the biogas system.

8. A method for producing biogas from renewable lignin-containing resources, which consist at least in part of straw, in a biogas plant (10) according to any of claims 1 to 7, wherein the method comprises the following steps:
introducing the lignin-containing renewable resources, which consist at least in part of straw, as a fermentation substrate into a garage-shaped fermenter which is suitable for a solid matter fermentation,
producing mesophilic conditions in the fermenter (16), which permit an anaerobic bacterial fermentation,
removing fermentation residues from the fermenter, and
producing fuel pellets or fuel briquettes by dehydrating and pelleting the fermentation residues.

9. The method according to claim 8, in which the straw is pretreated to effect a chemical, thermal and/or mechanical decomposition thereof and/or
at least 30 % by weight, preferably at least 50 % by weight, of the dry substance of the substrate used for fermentation preferably consists of straw or
in which the dry substance of the substrate used for fermentation contains preferably 30 % by weight to 90 % by weight of pure straw, 10 % by weight to 70 % by weight of solid manure and 0 % by weight to 30 % by weight of further substrates.

10. The method according to any of claims 8 or 9, in which materials harmful in combustion, in particular chlorine, nitrogen, sulfur, potassium chloride and/or silicates, are washed out of or eluted from the fermentation residues after their removal from the fermenter, wherein
the washing-out of the fermentation residues is preferably made in the fermentation residue bunker (60), and/or
the fermentation residues are mechanically dehydrated after the washing-out, in particular by means of a screw press (70) or a decanter, wherein the resulting liquid phase is recycled into the fermentation residue bunker (60) as process water, and/or
the fermentation residues are dried in a drying device, in particular a belt drier (142) or a drum drier with contact drying, preferably with waste heat from at least one gas engine of the biogas plant (10) and/or the at least in part dehydrated and optionally dried fermentation residues are mechanically comminuted, in particular in a hammer mill (146) or straw mill, and/or
the comminuted and at least in part dehydrated and/or dried fermentation residues are mixed with one or more of the following additions: cereal flour, molasses, caustic lime, steam, chipped wood, sawdust, oil press cake, calcium, magnesium, aluminum, nitrogen-binding agents, in particular nitrogen-reducing reductants, hydrated lime and/or the comminuted and dehydrated fermentation residues are pressed into pellets or briquettes.

11. The method according to any of claims 9 to 10, in which the pretreatment for thermal decomposition comprises a saturated steam treatment, wherein
the saturated steam treatment is preferably carried out in such a way that it softens lignin structures of the straw while the outer structure of the raw material is substantially maintained on the whole, and/or
the saturated steam treatment is carried out at a temperature between 160°C and 240°C and at a pressure between 20 and 30 bar for less than 20 minutes, and/or
the treatment pressure at the end of the saturated steam treatment is lowered by at least 80 % within five seconds, and/or
the straw is soaked prior to the saturated steam treatment, and/or
the straw is soaked in an acidic solution, in particular percolate, in an alkaline solution or in liquid manure after the saturated steam treatment, and/or
the straw is mechanically comminuted prior to its further use, in particular prior to the saturated steam treatment, and/or
the straw is provided as bales, wherein
the bales (72) preferably have a density of at least 200 kg/m³, preferably of at least 208 kg/m³, and/or
the bales (72) are layered as the lowermost layer in a fermenter (16) and/or
the bales are dissolved after layering them in the fermenter (16).

12. The method according to any of claims 9 to 11, in which the chemical pretreatment comprises mixing the straw with solid manure, liquid manure, percolate and/or percolate-containing fermentation mass, and/or
in which the chemical pretreatment comprises soaking straw in a water-acid solution, a water-lye solution percolate or liquid manure and/or the straw is defibered prior to its use as a fermentation substrate.

13. The method according to any of claims 9 to 12, in which the pretreatment of the straw for the mechanical decomposition comprises chopping and/or cutting at least a portion thereof, wherein
the straw is preferably cut and/or chopped to a particle length of less than 5 cm, preferably less than 2 cm, and/or
in which between 10 % and 70 % of the straw is cut and/or chopped, and/or
in which the pretreatment of the straw for mechanical decomposition comprises the grinding of at least a portion thereof, wherein
between 5 % and 100 %, preferably between 20 % and 65 %, of the straw are ground, and/or
the grinding degree of the straw corresponds to a screen size diameter of at most 8 mm, preferably a screen size diameter of at most 1 mm, and/or no additional acids, enzymes, fungi or yeasts are added to the straw from outside between the pre-treatment and the introduction into the fermenter for the anaerobic fermentation.

14. The method according to any of claims 8 to 13, in which, in a refreshment of the fermentation mass, part of the fermentation mass removed from the fermenter (16) is dehydrated by means of a press, in particular a screw press, and is mixed as inoculation mass with new fresh mass and then returned into the fermenter (16) as refreshed fermentation mass, and/or the fermentation residues removed from the fermenter (16) are subsequently fermented in a fermentation residue bunker (60), wherein the temperature in the fermentation residue bunker (60) is 50 to 58°C, and the fermentation residue bunker (60) is connected to the biogas system, wherein preferably enzymes are introduced into the fermentation residue bunker (60), said enzymes increase the efficiency of the subsequent fermentation.

15. The method according to any of claims 8 to 14, in which the washed-out or eluted, dehydrated and comminuted fermentation residues are mixed with an amount of molasses, caustic lime, hydrated lime, chipped wood, sawdust, calcium, magnesium and/or aluminum before they are pelletized such that the pellets or briquettes achieve the following fuel quality:
as components in the exhaust gas with a reference oxygen volume of 13 %: 4 g CO/m³, 150 mg dust/Nm³,
preferably as components in the exhaust gas at a reference oxygen volume of 11 %: 0.25 g CO/Nm³, 50 g dust/Nm³, 50 mg total C/Nm³, 500 mg NOₓ/Nm³, 2.0 g SO₂/Nm³, and
in particular preferably as components in the exhaust gas at a reference oxygen volume of 11 %: 0.25 g CO/Nm³, 20 g dust/Nm³, 50 mg total C/Nm³, 400 mg NOₓ/Nm³, 2.0 g SO₂/Nm³, and/or the fermentation residues are defibered and/or dedusted prior to pelletization.

## Revendications

1. Installation de biogaz (10) pour la production de biogaz à partir de matières premières renouvelables lignocellulosiques constituées au moins en partie de paille, comprenant:
une pluralité de cuves de fermentation en forme de garage (16) destinées à une fermentation bactérienne anaérobie des matières premières renouvelables lignocellulosiques constituées au moins en partie de paille, comme substrat de fermentation après le processus de fermentation de matières solides à un niveau de température mésophile, et un moyen (64) destiné à produire des pellets combustibles et/ou de briquettes combustibles par déshydratation et pelletisation des résidus de fermentation.

2. Installation de biogaz (10) selon la revendication 1, avec un moyen (82, 104, 122, 130) destiné à la désagrégation chimique, mécanique et/ou thermique des matières premières lignocellulosiques renouvelables constituées au moins en partie de paille,
dans lequel le moyen destiné à désagréger la paille comporte un moyen destiné à broyer mécaniquement la paille, en particulier un broyeur à marteaux ou un broyeur à paille, et/ou un moyen destiné au traitement à la vapeur saturée,
dans lequel le moyen (82, 104, 122, 130) destiné au traitement à la vapeur saturée est de préférence un conteneur sous pression (84) et comporte des moyens (112, 114) convenant pour générer dans le conteneur sous pression (84) une vapeur d'eau à une pression comprise entre 20 et 30 bar et une température comprise entre 180°C et 250°C et/ou comporte un conteneur pour le trempage de la paille dans de l'eau, une solution aqueuse alcaline, une solution aqueuse acide, un percolat ou un purin.

3. Installation de biogaz (10) selon la revendication 1 ou 2, avec un moyen (60) de lixiviation ou d'élution des résidus de fermentation, et/ou avec au moins un moteur à gaz et au moins un générateur de courant qui peut être actionné par le moteur à gaz.

4. Installation de biogaz (10) selon l'une des revendications précédentes, dans lequel le moyen (64) destiné à produire des pellets combustibles et/ou des briquettes combustibles comporte un moyen destiné à la déshydratation mécanique des résidus de fermentation, en particulier une presse à vis (70) ou un décanteur, et/ou un moyen de séchage, en particulier un séchoir à bande (142) ou un séchoir à tambour à séchage par contact, dans laquelle
le moyen de séchage (142) fonctionne de préférence à l'aide de la chaleur perdue d'au moins un moteur à gaz de l'installation de biogaz (10) et dans laquelle la température du moyen de séchage n'excède de préférence pas 180°C, de préférence pas 140°C.

5. Installation de biogaz (10) selon l'une des revendications précédentes, dans lequel le moyen (64) destiné à produire des pellets combustibles et/ou des briquettes combustibles comporte un moyen destiné à broyer les résidus de fermentation au moins partiellement déshydratés et éventuellement séchés, en particulier un broyeur à marteaux (146) ou un broyeur de paille, et/ou comporte un moyen de conditionnement (150) convenant pour mélanger aux résidus de fermentation broyés et au moins partiellement déshydratés et/ou séchés un ou plusieurs des additifs suivants: de la farine de céréales, de la mélasse, de la chaux vive, de la vapeur, des copeaux de bois, de la sciure de bois, des tourteaux, du calcium, du magnésium, de l'aluminium, des liants d'azote, en particulier, des agents réducteurs réduisant l'azote, de l'hydrate de chaux, et/ou comporte une presse de pelletisation (152) convenant pour presser les résidus de fermentation broyés et déshydratés pour obtenir des pellets ou des briquettes, dans laquelle
de préférence au moins certains des composants du moyen (64) destiné à la déshydratation et la pelletisation de résidus de fermentation sont connectés par un convoyeur pneumatique (144, 148, 154, 158), et/ou
avec une zone de livraison et de chargement (24), dans laquelle
le moyen destiné à la désagrégation chimique, mécanique et/ou thermique de paille est logé de préférence dans la zone de livraison et de chargement (24), et/ou dans laquelle la zone de livraison et de chargement (24) comporte de préférence un moyen technique de transport stationnaire (44, 46, 56) convenant pour transporter de la biomasse de la zone de livraison et de chargement (24) vers un parvis de fermentateurs (28) à partir duquel sont accessibles une pluralité de fermenteurs (16) du type garage, et/ou
dans laquelle la zone de livraison et de chargement (24) comporte au moins un bunker incorporé (42) pour la masse fraîche.

6. Installation de biogaz (10) selon la revendication 5, avec des premiers moyens de transport (44) convenant pour transporter de la masse fraîche de l'au moins un bunker de livraison (42) pour la masse fraîche vers un bunker de masse fraîche (46), dans laquelle
les premiers moyens de transport comportent de préférence une bande transporteuse (44) sur laquelle de la masse fraîche de différents bunkers de livraison (42) peut être transportée vers le bunker de masse fraîche (46), optionnellement avec des deuxièmes moyens de transport, en particulier une lame de poussée, convenant pour transporter la masse fraîche à travers le bunker de masse fraîche (46) en direction du parvis de fermenteurs (18), dans laquelle dans la zone de livraison et de chargement (24) est prévu, de préférence, un endroit de déchargement (50) avec une grue et un entrepôt intermédiaire pour le matériau en balles, en particulier de la paille, et dans laquelle, de préférence sont prévus des troisièmes moyens de transport, en particulier des convoyeurs à rouleaux ou convoyeurs pousseurs, convenant pour transporter des balles ou paquets de balles individuels le long d'un canal à balles (58) vers le parvis de fermenteurs (18).

7. Installation de biogaz (10) selon l'une des revendications précédentes, avec un bunker à résidus de fermentation (60) accessible depuis le parvis à fermentateurs (18) pour l'introduction de résidus de fermentation, dans laquelle le bunker à résidus de fermentation (60) comporte de préférence des moyens de transport stationnaires, en particulier des convoyeurs à vis convenant pour transporter les résidus de fermentation à travers le bunker de résidus de fermentation (60), dans laquelle le bunker de résidus de fermentation (60) est de préférence raccordé au système de biogaz.

8. Procédé de production de biogaz à partir de matières premières lignocellulosiques renouvelables constituées au moins en partie de paille, dans une installation de biogaz (10) selon l'une des revendications 1 à 7, le procédé présentant les étapes suivantes consistant à:
introduire les matières premières renouvelables lignocellulosiques, constituées au moins en partie de paille, comme substrat de fermentation dans un fermenteur en forme de garage convenant pour une fermentation de matières solides,
générer des conditions mésophiles dans le fermenteur (16) permettant une fermentation bactérienne anaérobie,
éliminer les résidus de fermentation du fermenteur, et
produire des pellets combustibles ou des briquettes combustibles par déshydratation et pelletisation des résidus de fermentation.

9. Procédé selon la revendication 8, dans lequel la paille est prétraitée pour provoquer une désagrégation chimique, thermique et/ou mécanique de cette dernière, et/ou
la substance sèche du substrat utilisé pour la fermentation est constituée de préférence d'au moins 30% en poids, de préférence d'au moins 50% en poids, de paille, ou
dans lequel la substance sèche du substrat utilisé pour la fermentation contient de préférence de 30% en poids à 90% en poids de paille pure, de 10% en poids à 70% en poids de fumier solide et de 0% en poids à 30% en poids d'autres substrats.

10. Procédé selon l'une des revendications 8 ou 9, dans lequel les substances nocives pour la combustion, en particulier le chlore, l'azote, le soufre, le chlorure de potassium et/ou les silicates, sont lixiviés ou élués des résidus de fermentation après leur élimination du fermenteur, dans lequel
la lixiviation des résidus de fermentation est réalisée de préférence dans le bunker de résidus de fermentation (60), et/ou
les résidus de fermentation sont déshydratés mécaniquement après la lixiviation, en particulier à l'aide d'une presse à vis (70) ou d'un décanteur, où la phase liquide résultante est réalimentée comme eau de processus vers le bunker de résidus de fermentation (60), et/ou
les résidus de fermentation sont séchés dans un moyen de séchage, en particulier un séchoir à bande (142) ou un séchoir à tambour par séchage par contact de préférence à l'aide de la chaleur perdue d'au moins un moteur à gaz de l'installation de biogaz (10) et/ou les résidus de fermentation au moins partiellement déshydratés et éventuellement séchés sont broyés mécaniquement, en particulier dans un broyeur à marteaux (146) ou broyeur à paille, et/ou
aux résidus de fermentation broyés et au moins partiellement déshydratés et/ou séchés sont ajoutés un ou plusieurs des additifs suivants: de la farine de céréales, de la mélasse, de la chaux vive, de la vapeur, des copeaux de bois, de la sciure de bois, des tourteaux, du calcium, du magnésium, de l'aluminium, des liants d'azote, en particulier, des agents réducteurs réduisant l'azote, de l'hydrate de chaux, et/ou
les résidus de fermentation broyés et déshydratés sont pressés pour obtenir des pellets ou des briquettes.

11. Procédé selon l'une des revendications 9 à 10, dans lequel le prétraitement pour la désagrégation thermique comporte un traitement à la vapeur saturée, dans lequel
le traitement à la vapeur saturée est réalisé de préférence de sorte qu'il ramollisse les structures de lignine de la paille, mais que la structure extérieure de la matière première persiste sensiblement dans son ensemble, et/ou
le traitement à la vapeur saturée est réalisé à une température comprise entre 160°C et 240°C et à une pression comprise entre 20 et 30 bars pendant moins de 20 minutes, et/ou
la pression de traitement à la fin du traitement à la vapeur saturée est réduite d'au moins 80% en cinq secondes, et/ou
la paille est trempée avant le traitement à la vapeur saturée, et/ou
la paille est trempée après le traitement à la vapeur saturée dans une solution acide, en particulier un percolat, dans une solution alcaline ou dans du purin, et/ou
la paille est broyée mécaniquement avant utilisation successive, en particulier avant le traitement à la vapeur saturée, et/ou
la paille est mise à disposition sous forme de balles, où
les balles (72) présentent de préférence une densité d'au moins 200 kg/m³, de préférence d'au moins 208 kg/m³, et/ou
les balles (72) sont mises en couche comme couche inférieure dans un fermenteur (16) et/ou
les balles sont dissoutes après avoir été mises en couche dans le fermenteur (16).

12. Procédé selon l'une des revendications 9 à 11, dans lequel le prétraitement chimique comporte un mélange de la paille avec du fumier solide, du purin, du percolat et/ou de la masse defermentation contenant du percolat, et/ou
dans lequel le prétraitement chimique comporte le trempage de paille dans une solution aqueuse d'acide, une solution aqueuse alcaline, du percolat ou du purin, et/ou la paille est défibrée avant utilisation comme substrat de fermentation.

13. Procédé selon l'une des revendications 9 à 12, dans lequel le prétraitement de la paille pour la désagrégation mécanique comporte le hachage et/ou la coupe d'au moins une part de cette dernière, dans lequel
la paille est de préférence coupée et/ou hachée à une longueur de particules de moins de 5 cm, de préférence de moins de 2 cm, et/ou
dans lequel une part comprise entre 10% et 70% de la paille est coupée et/ou hachée, et/ou
dans lequel le prétraitement de la paille pour la désagrégation mécanique comporte le broyage d'au moins une part de cette dernière, dans lequel
une part comprise entre 5% et 100%, de préférence entre 20% et 65% de la paille est broyée, et/ou
le degré de broyage de la paille correspond à un diamètre de maille de tamis de tout au plus 8 mm, de préférence à un diamètre de maille de tamis de tout au plus 1 mm, et/ou
il n'est pas ajouté d'acides, d'enzymes, de champignons ou de levures additionnels de l'extérieur à la paille entre le prétraitement et l'introduction dans le fermenteur pour la fermentation anaérobie.

14. Procédé selon l'une des revendications 8 à 13, dans lequel, lors d'un rafraîchissement de la masse de fermentation, une partie de la masse de fermentation prélevée du fermenteur (16) est déshydratée à l'aide d'une presse, en particulier d'une presse à vis, et mélangée comme inoculum avec une nouvelle masse fraîche et ensuite réintroduite comme masse de fermentation raffraîchie dans le fermenteur (16), et/ou les résidus de fermentation prélevés du fermenteur (16) sont post-fermentés dans un bunker de résidus de fermentation (60), dans lequel la température dans le bunker de résidus de fermentation (60) est de 50 à 58°C, et le bunker de résidus de fermentation (60) est raccordée au système de biogaz, dans lequel il est de préférence introduit des enzymes dans le bunker de résidus de fermentation (60) qui augmentent l'efficacité de la post-fermentation.

15. Procédé selon l'une des revendications 8 à 14, dans lequel il est mélangé avec les résidus de fermentation déshydratés et broyés lixiviés ou élués, avant leur pelletisation, tant de mélasse, de chaux vive, d'hydrate de chaux, de copeaux de bois, de sciure de bois, de calcium, de magnésium et/ou d'aluminium que les pellets ou les briquettes atteignent la qualité de combustible suivante:
comme composants dans le gaz d'échappement à un volume d'oxygène de référence de 13%: 4 g de CO/m³, 150 mg de poussière/Nm³,
de préférence comme composants dans le gaz d'échappement à un volume d'oxygène de référence de 11%: 0,25 g de CO/Nm³, 50 g de poussière/Nm³, 50 mg de C total/Nm³, 500 mg de NOₓ,/Nm³, 2,0 g de SO₂/Nm³, et
en particulier de préférence comme composants dans le gaz d'échappement à un volume d'oxygène de référence de 11%: 0,25 g de CO/Nm³, 20 g de poussière/Nm³, 50 mg de C total/Nm³, 400 mg de NOₓ/Nm³, 2,0 g de SO₂/Nm³, et/ou les résidus de fermentation sont défibrés et/ou dépoussiérés avant la pelletisation.
